# EUROPEAN PATENT APPLICATION

(11) **EP 4 287 194 A1**
(43) Date of publication of application: **06.12.2023**
(21) Application number: 23176019.0
(22) Date of filing: 30.05.2023
(51) Int. Cl.: G16H 10/40, G16H 20/40, G16H 40/67

(54) **MANAGEMENT METHOD, INFORMATION PROCESSING APPARATUS**

(30) Priority: 03.06.2022 JP 2022091168
(71) Applicant: SYSMEX CORPORATION, Kobe-shi Hyogo 651-0073 (JP)
(72) Inventor: Shirakami, Atsushi, Kobe-shi, Hyogo, 651-0073 (JP); Fukuma, Daigo, Kobe-shi, Hyogo, 651-0073 (JP)
(74) Representative: Hoffmann Eitle

(57) **Abstract**

Disclosed is a method for managing information on a blood product with a computer, including: obtaining a measurement result of a specimen, collected from the blood product, which is measured by a blood analyzer with product identification information of the blood product being used as specimen identification information of the specimen, and the specimen identification information; and in a database in which information on the blood product is stored in association with the product identification information, associating the measurement result with other information on the blood product other than the measurement result, on the basis of the product identification information used as the specimen identification information.

## Description

### CROSS REFERENCE TO RELATED APPLICATIONS

This application claims priority from prior Japanese Patent Application No. 2022-091168, filed on June 03, 2022, entitled "MANAGEMENT METHOD, INFORMATION PROCESSING APPARATUS, INFORMATION MANAGEMENT SYSTEM, AND BLOOD ANALYZER", the entire content of which is incorporated herein by reference.

### FIELD OF THE INVENTION

The present invention relates to a management method, and an information processing apparatus.

### BACKGROUND OF THE INVENTION

Blood products are pharmaceutical products including, as active ingredients, specific components separated from blood. Blood of donors collected in blood collection facilities such as blood donation rooms and bloodmobiles is transported to a factory, and blood products are produced in the factory through processes such as removal of white blood cells and platelets, centrifugation, and the like. The blood products produced in the factory are subjected to measurement of component quantities in the blood products for the purpose of confirming the quality. This measurement can be performed using a blood analyzer described in Japanese Laid-Open Patent Publication No. 2019-35620, for example. Thereafter, the blood products are shipped to medical institutions, and are transfused into patients undergoing surgical operations, patients affected by blood diseases and cancers, and the like.

Japanese Laid-Open Patent Publication No. 2004-94621 discloses an information management system. In this system, information, such as donor information, production history, distribution history, and storage history, of a produced blood product is recorded on a management IC tag attached to a blood bag that contains the blood product. When the blood product in the blood bag is used for transfusion, the information recorded on the management IC tag is read, transmitted to a management server, and managed in the management server, thereby improving traceability of the blood product.

### SUMMARY OF THE INVENTION

The measurement result of component quantities in a produced blood product is important information for managing the quality of the blood product, and is required to be unitarily managed together with other information on the blood product other than the measurement result. Japanese Laid-Open Patent Publication No. 2019-35620 discloses measurement of component quantities in a blood product, but does not disclose unitary management of the measurement result of component quantities in the blood product together with information of the blood product other than the measurement result. Meanwhile, Japanese Laid-Open Patent Publication No. 2004-94621 discloses management, in the management server, of the information such as donor information, production history, distribution history, and storage history, but does not disclose unitary management of the measurement result of component quantities in a blood product together with information of the blood product other than the measurement result.

In view of the above problems, an object of the present invention is to provide a management method, an information processing apparatus, an information management system, and a blood analyzer capable of unitarily managing a measurement result of a blood product and other information on the blood product other than the measurement result.

A method for managing information on a blood product with a computer (2), according to the present invention, includes: obtaining a measurement result of a specimen, collected from the blood product, which is measured by a blood analyzer (1, 7) with product identification information of the blood product being used as specimen identification information of the specimen, and the specimen identification information (S23); and in a database (2a) in which information on the blood product is stored in association with the product identification information, associating the measurement result with other information on the blood product other than the measurement result, on the basis of the product identification information used as the specimen identification information (S24).

According to the management method of the present invention, by using the product identification information of the blood product as the specimen identification information of the specimen collected from the blood product, the specimen identification information of the specimen and the measurement result of the specimen measured by the blood analyzer are obtained, and the obtained measurement result and the other information on the blood product can be associated with the product identification information. This enables unitary management of the measurement result of the specimen measured by the blood analyzer and the other information on the blood product.

An information processing apparatus (2) of the present invention includes a storage unit (202), and a controller (201) programmed to store information on a blood product into the storage unit (202). The controller (201) is programmed to: obtain, from a blood analyzer (1), a measurement result of a specimen, collected from the blood product, which is measured with product identification information of the blood product being used as specimen identification information of the specimen, and the specimen identification information; and in a database (2a), in the storage unit (202), in which information on the blood product is stored in association with the product identification information, associate the measurement result with other information on the blood product other than the measurement result, on the basis of the product identification information used as the specimen identification information.

According to the information processing apparatus of the present invention, by using the product identification information of the blood product as the specimen identification information of the specimen collected from the blood product, the specimen identification information of the specimen and the measurement result of the specimen measured by the blood analyzer are obtained, and the obtained measurement result and the other information on the blood product can be associated with the product identification information. This enables unitary management of the measurement result of the specimen measured by the blood analyzer and the other information on the blood product.

An information management system (3) of the present invention includes: a blood analyzer (1) capable of measuring a specimen collected from a blood product, with product identification information of the blood product being used as specimen identification information of the specimen; and an information processing apparatus (2) communicably connected to the blood analyzer (1). The information processing apparatus (2) includes a storage unit (202), and a controller (201) programmed to store information on the blood product into the storage unit (202). The controller (201) is programmed to: obtain, from the blood analyzer (1) , a measurement result of the specimen and the specimen identification information; and in a database (2a), in the storage unit (202), in which information on the blood product is stored in association with the product identification information, associate the measurement result with other information on the blood product other than the measurement result, on the basis of the product identification information used as the specimen identification information.

According to the information management system of the present invention, by using the product identification information of the blood product as the specimen identification information of the specimen collected from the blood product, the specimen identification information of the specimen and the measurement result of the specimen measured by the blood analyzer are obtained, and the obtained measurement result and the other information on the blood product can be associated with the product identification information. This enables unitary management of the measurement result of the specimen measured by the blood analyzer and the other information on the blood product.

An information management system (3) of the present invention includes: a blood analyzer (1) configured to measure a specimen collected from a blood product; and an information processing apparatus (2) communicably connected to the blood analyzer (1). The blood analyzer (1) obtains product identification information of the blood product, measures the specimen, and automatically transmits a measurement result of the specimen and the product identification information of the blood product to the information processing apparatus (2) (S 14). The information processing apparatus (2) includes a storage unit (202), and a controller (201) programmed to store information on the blood product into the storage unit (202). The controller (201) is programmed to: receive, from the blood analyzer (1), the measurement result of the specimen and the product identification information of the blood product (S23); and in a database (2a), in the storage unit (202), in which the information on the blood product is stored in association with the product identification information, automatically associate the received measurement result with other information on the blood product other than the measurement result, on the basis of the received product identification information (S24).

According to the information management system of the present invention, the measurement result of the blood product measured by the blood analyzer and the other information on the blood product can be unitarily managed in association with each other in the database. Moreover, since transmission of the measurement result and the product identification information from the blood analyzer and association of the measurement result of the blood product with the other information on the blood product in the database, are automatically performed, unitary management of these pieces of information can be easily and appropriately performed.

A blood analyzer (7) of the present invention includes: a detector (115) configured to detect a predetermined component from a specimen collected from a blood product; a reader (113) configured to read product identification information of the blood product as specimen identification information of the specimen; and a storage unit (122). The blood analyzer (7) is configured to: generate a measurement result of the specimen on the basis of a detection result of the detector (115); and in a database (2a), in the storage unit (122), in which information on the blood product is stored in association with the product identification information, associate the measurement result with other information on the blood product other than the measurement result, on the basis of the product identification information used as the specimen identification information (S24).

According to the blood analyzer of the present invention, by using the product identification information of the blood product as the specimen identification information of the specimen collected from the blood product, the specimen identification information of the specimen is read, the measurement result of the specimen is generated, and the generated measurement result and the other information on the blood product can be associated with the product identification information. This enables unitary management of the measurement result of the specimen and the other information on the blood product.

According to the present invention, a measurement result of a blood product and other information on the blood product other than the measurement result can be unitarily managed.

### BRIEF DESCRIPTION OF THE DRAWINGS

FIG. 1 schematically shows a series of processes including blood collection, production of blood products, and testing, according to Embodiment 1;
FIG. 2 schematically shows a barcode label sheet printed by a barcode printer, according to Embodiment 1;
FIG. 3 schematically shows connection of devices, according to a modification of Embodiment 1;
FIG. 4 schematically shows items stored in a database, according to Embodiment 1;
FIG. 5 is a block diagram showing a configuration of a blood analyzer, according to Embodiment 1;
FIG. 6 is a block diagram showing a configuration of an information processing apparatus, according to Embodiment 1;
FIG. 7 is a flowchart showing a process performed by the blood analyzer, according to Embodiment 1;
FIG. 8 is a flowchart showing a process performed by the information processing apparatus, according to Embodiment 1;
FIG. 9 schematically shows a screen displaying, in time series, statistical results obtained by aggregating measurement results, according to Embodiment 1;
FIG. 10 schematically shows a screen displaying, in time series, the statistical results obtained by aggregating the measurement results, according to Embodiment 1;
FIG. 11 schematically shows a screen displaying, for each period, the statistical results obtained by aggregating the measurement results, according to Embodiment 1;
FIG. 12 schematically shows a screen for inputting an instruction to extract and display measurement results, according to Embodiment 1;
FIG. 13 schematically shows a series of processes including blood collection, production of blood products, and testing, according to Modification 1 of Embodiment 1;
FIG. 14 schematically shows a screen, for inputting a measurement order, which is displayed on a display of the blood analyzer, according to Modification 1 of Embodiment 1;
FIG. 15 schematically shows a series of processes including blood collection, production of blood products, and testing, according to Modification 2 of Embodiment 1;
FIG. 16 schematically shows items stored in the database, according to Modification 2 of Embodiment 1;
FIG. 17 schematically shows a series of processes including blood collection, production of blood products, and testing, according to Embodiment 2;
FIG. 18 schematically shows a series of processes including blood collection, production of blood products, and testing, according to Embodiment 3;
FIG. 19 schematically shows connection of devices, according to Embodiment 3;
FIG. 20 schematically shows items stored in the database, according to Embodiment 3;
FIG. 21 schematically shows a screen displaying individual measurement results, according to Embodiment 3;
FIG. 22 schematically shows a screen displaying, in time series, statistical results obtained by aggregating measurement results, according to Embodiment 3;
FIG. 23 schematically shows items stored in the database, according to Embodiment 4;
FIG. 24 schematically shows an example in which quality control is performed in each production cycle and in each measurement cycle, according to Embodiment 4;
FIG. 25 schematically shows a screen for manually generating a group, according to Embodiment 4;
FIG. 26 is a flowchart showing a grouping process by the information processing apparatus, according to Embodiment 4;
FIG. 27 is a flowchart showing a display process for each group by the information processing apparatus, according to Embodiment 4;
FIG. 28 schematically shows a screen displaying quality control information based on the measurement results of blood products included in a group, according to Embodiment 4;
FIG. 29 schematically shows a screen displaying quality control information based on the measurement results of blood products included in a group, according to Embodiment 4;
FIG. 30 schematically shows a screen displaying, for each device ID, quality control information based on the measurement results of blood products included in a group, according to Embodiment 4;
FIG. 31 schematically shows a screen displaying, according to a filter condition, quality control information based on the measurement results of blood products included in a group, according to Embodiment 4;
FIG. 32 shows an example in which quality control information is associated with product identification information, according to Embodiment 5;
FIG. 33 shows an example in which group IDs are associated with quality control information, according to Embodiment 5;
FIG. 34 schematically shows a screen displaying information on blood products and a screen displaying groups of blood products, according to Embodiment 5;
FIG. 35 is a flowchart showing a process performed by the blood analyzer, according to Embodiment 5;
FIG. 36 is a flowchart showing a process performed by the information processing apparatus, according to Embodiment 5;
FIG. 37 schematically shows a screen for displaying a list of quality control information, according to Embodiment 5;
FIG. 38 schematically shows a screen showing details of quality control information, according to Embodiment 5;
FIG. 39 schematically shows a screen for confirming the number of pieces of approved quality control information associated with a group ID, according to Embodiment 5;
FIG. 40 schematically shows connection of devices, according to Embodiment 6;
FIG. 41 is a block diagram showing a configuration of an intermediate server, according to Embodiment 6;
FIG. 42 schematically shows items stored in the database, according to Embodiment 6;
FIG. 43 schematically shows items stored in the database, according to a modification of Embodiment 6;
FIG. 44 is a block diagram showing a configuration of a blood analyzer, according to Embodiment 7; and
FIG. 45 is a flowchart showing a process performed by the blood analyzer, according to Embodiment 7.

### DETAILED DESCRIPTION

Embodiments described below are obtained by applying the present disclosure to a management method, an information processing apparatus, an information management system, and a blood analyzer that manage information on blood products produced from blood of donors. In the embodiments, blood products are component products that are prepared by separating red blood cells, platelets, or plasma from human blood.

### <Embodiment 1>

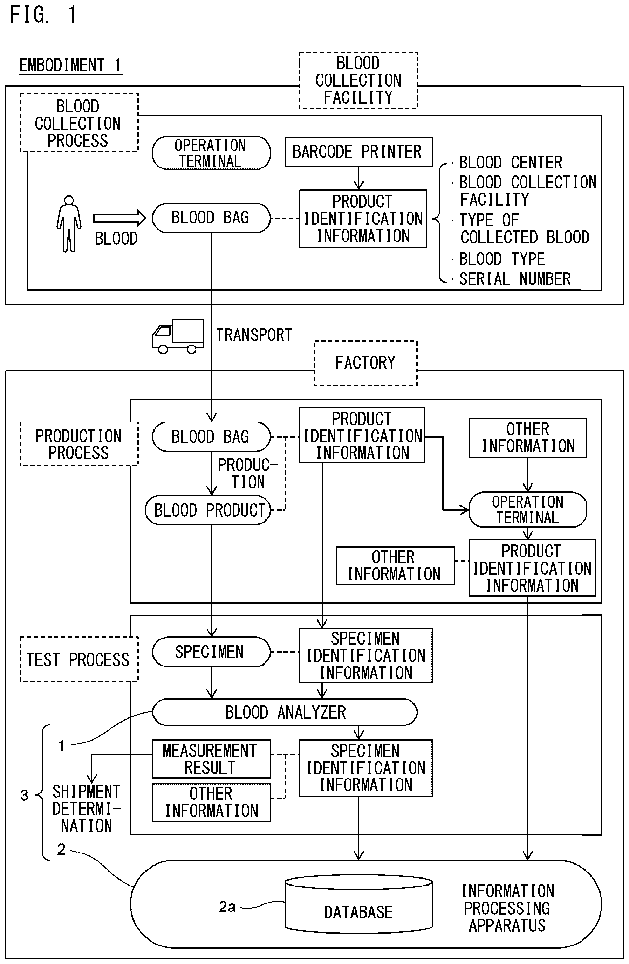

FIG. 1 schematically shows a series of processes including blood collection, production of blood products, and testing, according to Embodiment 1.

In a blood collection process, blood is collected from a donor in a blood collection facility such as a blood donation room or a bloodmobile. There are three types of blood collections, i.e., whole blood collection of collecting 200 mL of whole blood, whole blood collection of collecting 400 mL of whole blood, and blood component collection of collecting only platelets. When blood to be collected is whole blood, collected whole blood is stored in a blood bag as it is. When blood to be collected is platelet components, only platelet components are collected from the donor in the blood collection facility by using a blood component collection apparatus, and the collected platelet components are stored in a blood bag together with a blood preservation solution.

Each blood bag is given product identification information that allows the blood stored in the blood bag to be individually identified.

Product identification information is obtained as follows. That is, a blood center, a blood collection facility, the type of collected blood, and a blood type are respectively encoded into numbers, the numbers are reconstructed into a combined number, and a serial number is assigned to the combined number. For example, in the case of Japan, the number obtained by encoding the blood center is a number for identifying one of a plurality of blood centers existing in Japan. As the number obtained by encoding the blood center, the number of the blood center to which the blood collection facility belongs is used. The number obtained by encoding the blood collection facility is identification information for identifying a blood collection facility such as a bloodmobile or a blood donation room where blood collection was performed, for example. The number obtained by encoding the type of collected blood is a number indicating any one of 200 mL of whole blood, 400 mL of whole blood, and platelet components. The number obtained by encoding the blood type is a number indicating any one of A, O, B, and AB. The serial number is a serially assigned number having a predetermined number of digits, and makes the product identification information individually identifiable.

For example, the numbers obtained by encoding the blood center, the blood collection facility, the type of collected blood, and the blood type are "37", "21", "3" (platelet components), and "4" (O), respectively, and the serial number is "0636". In this case, the product identification information is "37-2134-0636". The product identification information may not necessarily be information obtained by encoding the blood center, the blood collection facility, and the like. For example, only the serial number may be used as the product identification information.

In the blood collection facility, an operation terminal and a barcode printer connected to the operation terminal are installed. An operator of the blood collection facility inputs product identification information determined based on the above rule into the operation terminal, and instructs the barcode printer to print a barcode label sheet.

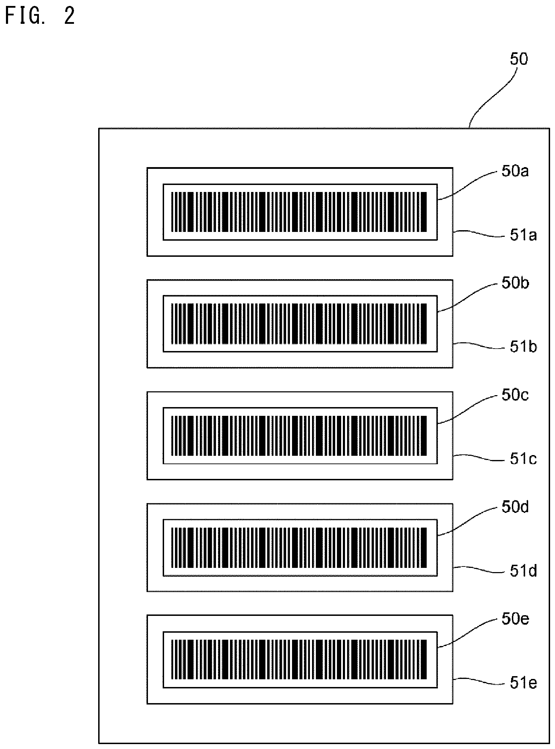

FIG. 2 schematically shows a barcode label sheet 50 printed by the barcode printer.

The barcode label sheet 50 is a sticker-like sheet with an adhesive applied to the entire back surface thereof, and the operator can peel off it. The barcode label sheet 50 includes five barcode labels 50a, 50b, 50c, 50d, and 50e. On the barcode labels 50a, 50b, 50c, 50d, and 50e, barcodes indicating the same product identification information are printed. The barcode labels 50a, 50b, 50c, 50d, and 50e have parting lines 51a, 51b, 51c, 51d, and 51e, respectively, at the outer peripheries thereof, so that the barcode labels 50a, 50b, 50c, 50d, and 50e can be removed from the barcode label sheet 50.

Referring back to FIG. 1, the barcode label sheet 50 is attached to the blood bag. Thereafter, the blood bag is transported to a factory.

In the factory, a production process and a test process are performed. A blood product is produced in the production process, and the produced blood product is tested in the test process. In the factory, a blood analyzer 1 and an information processing apparatus 2 are installed. The blood analyzer 1 and the information processing apparatus 2 constitute an information management system 3.

Although the information management system 3 is installed in the factory in the example shown in FIG. 1, the information management system 3 may be installed in a medical institution such as a hospital where transfusion is performed, and may be used for quality control for blood products before transfusion.

In the production process, when the blood in the blood bag is whole blood, the operator removes white blood cells and platelets from the whole blood in the blood bag by using a white blood cell removal filter. Then, the operator separates red blood cells and plasma from each other in the blood after the removal process by using a centrifuge, and stores the obtained red blood cells and plasma into different blood bags together with blood preservation solutions. The operator peels off four labels out of the barcode labels 50a, 50b, 50c, 50d, and 50e from the blood bag in which the whole blood has been stored, and puts two labels on each of the blood bag for a red blood cell product and the blood bag for a plasma product. Thus, a red blood cell product of extracted red blood cells and a plasma product of extracted plasma are produced. When the blood in the blood bag is platelet components, the platelet components in the blood bag become a platelet product.

In the production process, the operator operates the operation terminal to store, in a database 2a of the information processing apparatus 2, other information on the blood product in association with the product identification information. The other information on the blood product is information on the blood product other than the measurement results obtained in the test process. For example, the other information includes pieces of information respectively indicating blood center, blood collection facility, type of collected blood, blood type, reception date/time, volume, weight, result of visual inspection, product type, production date/time, factory, manufacturer, production device, and the like.

The blood center, the blood collection facility, the type of collected blood, and the blood type are obtained from the product identification information. The reception date/time is when the factory received the blood bag. The volume, the weight, and the result of visual inspection are the results of measurement and inspection performed by the operator when the blood bag was received. The product type is the type of the blood product, and is information indicating any of a red blood cell product, a plasma product, and a platelet product. The production date/time is when the blood product was produced. In the case of a red blood cell product, the production date/time is when the red blood cell product was produced in the production process. In the case of a plasma product obtained from whole blood, the production date/time is when the plasma product was produced in the production process. In the case where collected platelet components become a platelet product, the production date/time is when the blood component collection was performed in the blood collection process. The information indicating the factory is a factory ID for identifying the factory where the blood product was produced. The information indicating the manufacturer is a manufacturer ID for identifying the operator who produced the blood product. The information indicating the production device is a production device ID indicating the lot number of the white blood cell removal filter used in the production process, identification information of the centrifuge used in the production process, identification information of the blood component collection apparatus used in the blood component collection, or the like.

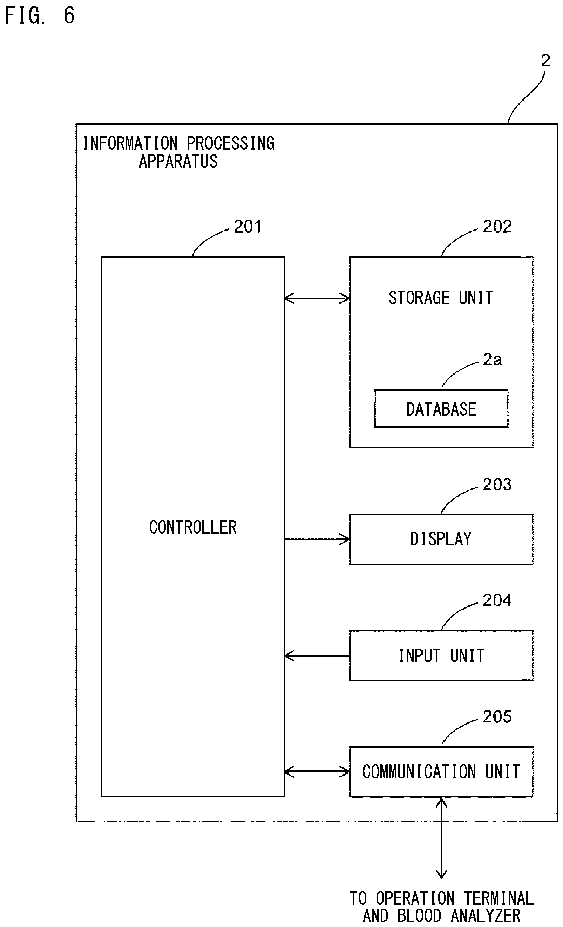

The operator may not necessarily perform storage of the product identification information and the other information on the blood product in the production process by operating the operation terminal. For example, the operator may store, in the database 2a, the product identification information and the other information on the blood product by operating an input unit 204 (see FIG. 6) of the information processing apparatus 2.

In the test process, the produced blood products are subjected to complete survey or sampling survey. The operator dispenses a part of the blood product as a specimen from the blood bag into a specimen container. The operator peels off any one of the barcode labels 50a, 50b, 50c, 50d, and 50e from the blood bag, and puts the label on the specimen container. The blood analyzer 1 is used for measurement and analysis of the specimen dispensed into the specimen container. The blood analyzer 1 is a device capable of analyzing blood cell components in blood specimens. For example, XN-Series Blood Bank mode, which is an automatic multi-item blood cell analyzer manufactured by SYSMEX CORPORATION, can be used. In the test process, the product identification information indicated by the barcode label attached to the specimen container is used as specimen identification information. The blood analyzer 1 reads the specimen identification information from the barcode label attached to the specimen container, measures the specimen in the specimen container, and stores the measurement result in association with the specimen identification information. The measurement result includes, for example, values of measurement items (red blood cell count, white blood cell count, platelet count, etc.), and a determination result as to whether the specimen is "normal" or "abnormal".

The blood analyzer 1 transmits the specimen identification information and the measurement result to the information processing apparatus 2 together with the measurement date/time and a device ID for identifying the blood analyzer 1. The measurement date/time and the device ID are pieces of other information on the blood product other than the measurement result.

The information processing apparatus 2 includes the database 2a in which information on each blood product is stored in association with product identification information. In the database 2a, the measurement result received from the blood analyzer 1 and the other information on the blood product other than the measurement result are associated with each other based on the product identification information. In Embodiment 1, since the specimen identification information is the same as the product identification information as described above, the measurement result and the other information on the blood product other than the measurement result can be associated with each other based on the product identification information in the database 2a. This enables unitary management of the specimen measurement result obtained by the blood analyzer 1 and the other information on the blood product. Therefore, the measurement result of the blood product measured for quality confirmation can be smoothly confirmed while improving traceability of the blood product.

Determination as to whether or not the blood product can be shipped, i.e., shipment determination, is performed based on the information on the blood product associated with the product identification information. When the determination result is that the blood product can be shipped, the blood product is shipped or stored for shipment.

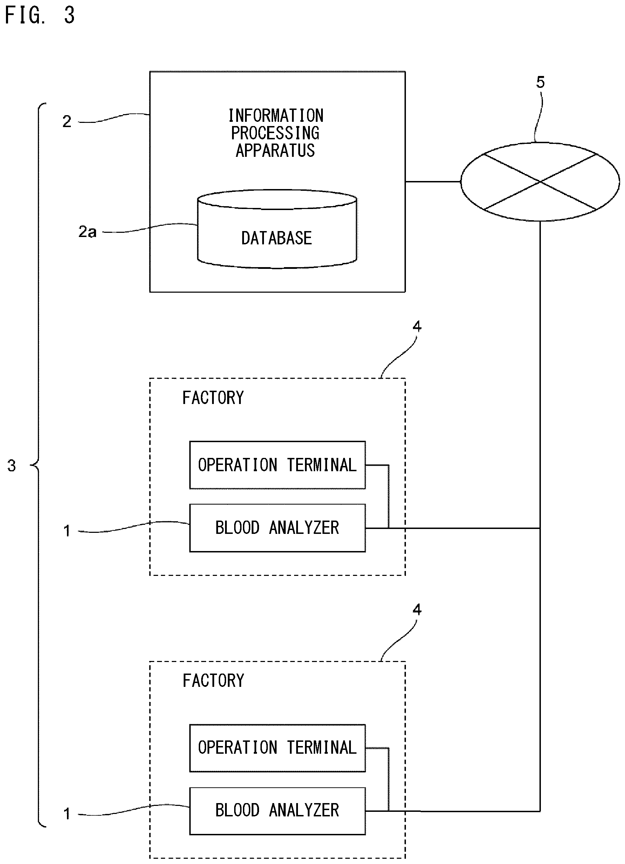

In FIG. 1, both the blood analyzer 1 and the information processing apparatus 2 are installed in the factory. However, as shown in FIG. 3, a blood analyzer 1 may be installed in each of a plurality of factories 4, and the blood analyzer 1 in each factory may be connected to an information processing apparatus 2 via a network 5 such as the Internet. An operation terminal is also installed in each factory 4. Each operation terminal is connected to the information processing apparatus 2 via the network 5 such as the Internet. In this case, storage of the product identification information and the other information into the database 2a in the production process is performed by the operation terminal installed in each factory 4.

In FIG. 1, the information processing apparatus 2 directly receives, from the blood analyzer 1, the measurement result and the other information on the blood product other than the measurement result transmitted from the blood analyzer 1, but may receive these pieces of information via another device.

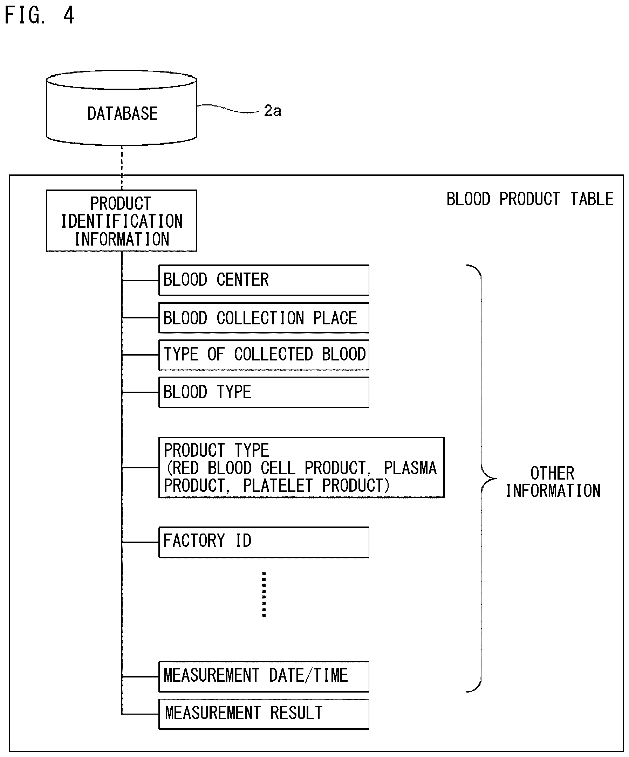

FIG. 4 schematically shows items stored in the database 2a.

The database 2a includes a blood product table containing items such as product identification information, blood center, blood collection facility, type of collected blood, blood type, product type, factory ID, measurement result, measurement date/time, and the like. On the blood product table, the measurement result and the other information on the blood product other than the measurement result are associated with each other based on the product identification information.

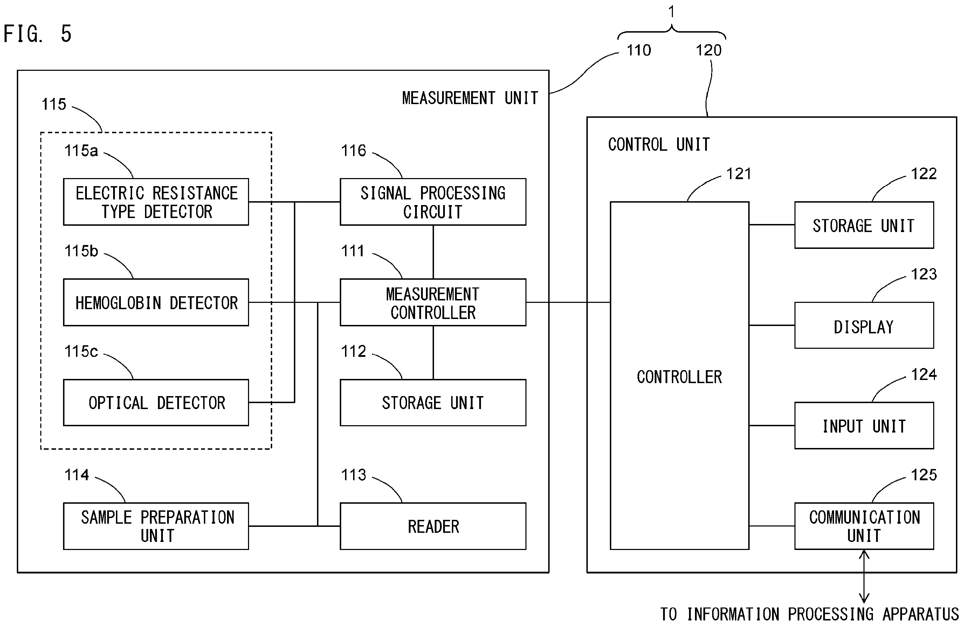

FIG. 5 is a block diagram showing the configuration of the blood analyzer 1.

The blood analyzer 1 includes a measurement unit 110 and a control unit 120.

The measurement unit 110 includes a measurement controller 111, a storage unit 112, a reader 113, a sample preparation unit 114, a detector 115, and a signal processing circuit 116.

The measurement controller 111 is implemented by an FPGA or a CPU, for example. The storage unit 112 is implemented by a ROM, a RAM, an SSD, an HDD, or the like, for example. The measurement controller 111 receives signals outputted from the components of the measurement unit 110, and controls the components of the measurement unit 110. The measurement controller 111 communicates with the control unit 120. The measurement controller 111 performs various kinds of processing based on a program stored in the storage unit 112.

The reader 113 is implemented by a barcode reader, for example. The reader 113 reads a barcode from a barcode label attached to a specimen container supplied to the blood analyzer 1, and obtains specimen identification information. A measurement order is set in advance on the specimen in association with the specimen identification information, and the measurement order includes information indicating which measurement items are the subject of measurement and analysis. For example, in the case of the measurement order corresponding to a red blood cell product, the measurement items include red blood cell count, residual white blood cell count, and the like. In the case of the measurement order corresponding to a plasma product, the measurement items include a residual red blood cell count, a residual white blood cell count, and the like. In the case of the measurement order corresponding to a platelet product, the measurement items include residual red blood cell count, residual white blood cell count, platelet count, and the like.

The sample preparation unit 114 aspirates the specimen from the specimen container, and mixes the aspirated specimen with a reagent to prepare a measurement sample corresponding to the measurement order. The sample preparation unit 114 mixes the specimen with predetermined reagents to prepare a red blood cell count/platelet count measurement sample, a hemoglobin measurement sample, a white blood cell count measurement sample, a white blood cell classification measurement sample, and a platelet count measurement sample.

The detector 115 detects predetermined components from the measurement samples prepared by the sample preparation unit 114. The detector 115 includes an electric resistance type detector 115a, a hemoglobin detector 115b, and an optical detector 115c. The electric resistance type detector 115a measures blood cells by a sheath flow DC detection method. The hemoglobin detector 115b measures hemoglobin by an SLS-hemoglobin method. The optical detector 115c measures blood cells by a flow cytometry method.

The signal processing circuit 116 outputs, as measurement data, a result of signal processing performed on detection signals respectively outputted from the electric resistance type detector 115a, the hemoglobin detector 115b, and the optical detector 115c, to the measurement controller 111.

The measurement controller 111 stores the measurement data outputted from the signal processing circuit 116 into the storage unit 112. When measurement for one specimen has ended, the measurement controller 111 transmits, to the control unit 120, the measurement data stored in the storage unit 112 in association with the specimen identification information read by the reader 113.

The control unit 120 includes a controller 121, a storage unit 122, a display 123, an input unit 124, and a communication unit 125.

The controller 121 is implemented by a CPU, for example. The storage unit 122 is implemented by an SSD or an HDD, for example. The controller 121 receives signals outputted from the components of the control unit 120, and controls the components of the control unit 124. The controller 121 communicates with the measurement controller 111 of the measurement unit 110, and controls the components of the measurement unit 110 via the measurement controller 111. The controller 121 stores the measurement data received from the measurement unit 110 into the storage unit 122. The controller 121 analyzes the specimen by using the measurement data on the basis of the program stored in the storage unit 122, thereby generating a measurement result. The controller 121 stores, in the storage unit 122, the generated measurement result in association with the specimen identification information.

For example, when the specimen is a red blood cell product, the controller 121 calculates a red blood cell count (RBC) per unit volume and a residual white blood cell count (WBC) per unit volume, from the measurement data obtained from the red blood cell count/platelet count measurement sample and the white blood cell classification measurement sample, respectively. When the specimen is a plasma product, the controller 121 calculates a residual white blood cell count (WBC) per unit volume and a residual red blood cell count (RBC) per unit volume, from the measurement data obtained from the white blood cell classification measurement sample and the red blood cell count/platelet count measurement sample, respectively. When the specimen is a platelet product, the controller 121 calculates a platelet count (PLT) per unit volume, a residual white blood cell count (WBC) per unit volume, and a residual red blood cell count (RBC) per unit volume, from the measurement data obtained from the platelet count measurement sample, the white blood cell classification measurement sample, and the red blood cell count/platelet count measurement sample, respectively. Thus, the controller 121 obtains the value of each measurement item as the measurement result.

The controller 121 determines whether each specimen is normal or abnormal based on the values of the measurement items. The phrase "specimen is normal" means that the blood product is acceptable as a product and can be shipped. The phrase "specimen is abnormal" means that the blood product is not acceptable as a product and cannot be shipped. The red blood cell product is determined to be normal when the red blood cell count is within a normal range and the residual white blood cell count is less than a predetermined value, for example. The plasma product is determined to be normal when the residual white blood cell count is less than the predetermined value and the residual red blood cell count is less than a predetermined value, for example. The platelet product is determined to be normal when the platelet count is within a normal range, the residual white blood cell count is less than the predetermined value, and the residual red blood cell count is less than the predetermined value, for example.

The display 123 is implemented by a liquid crystal display, for example. The display 123 displays various screens composed of images. For example, on the display 123, a screen indicating the measurement result for each specimen identification information is displayed. The input unit 124 is implemented by a keyboard and a mouse, for example. The operator inputs an instruction to the control unit 120 via the input unit 124.

The display 123 and the input unit 124 may be integrated with each other. For example, the control unit 120 may include a touch-panel type display, as the display 123 and the input unit 124.

The communication unit 125 includes a communication interface that is communicable with the information processing apparatus 2 on the basis of Ethernet standards, for example. The controller 121 transmits the measurement result and the measurement date/time in association with the specimen identification information, to the information processing apparatus 2 via the communication unit 125.

The measurement unit 110 and the control unit 120 may be integrated with each other. In this case, for example, the measurement controller 111 is omitted, and the controller 121 controls the components of the blood analyzer 1. For example, a device disclosed in U.S. Patent Publication No. 2019-0049383 can be used as the blood analyzer 1, and U.S. Patent Publication No. 2019-0049383 is hereby incorporated by reference.

FIG. 6 is a block diagram showing the configuration of the information processing apparatus 2.

The information processing apparatus 2 includes a controller 201, a storage unit 202, a display 203, an input unit 204, and a communication unit 205.

The controller 201 is implemented by a CPU, for example. The controller 201 receives signals outputted from the components of the information processing apparatus 2, and controls the components of the information processing apparatus 2. The storage unit 202 is implemented by an SSD, an HDD, or the like, for example. The storage unit 202 has the database 2a stored therein.

The display 203 is implemented by a liquid crystal display, for example. The display 203 displays various types of screens composed of images. The input unit 204 is implemented by a keyboard and a mouse, for example.

The display 203 and the input unit 204 may be integrated with each other. For example, the information processing apparatus 2 may include a touch-panel type display, as the display 203 and the input unit 204.

The communication unit 205 includes a communication interface that is communicable with the blood analyzer 1 and the operation terminal on the basis of Ethernet standards. Through the communication unit 205, the controller 201 receives the product identification information and the other information that have been inputted to the operation terminal in the production process, and receives the specimen identification information (product identification information), the measurement result, and the other information from the blood analyzer 1 in the test process. The controller 201 stores, in the database 2a, the received information on the blood product in association with the product identification information.

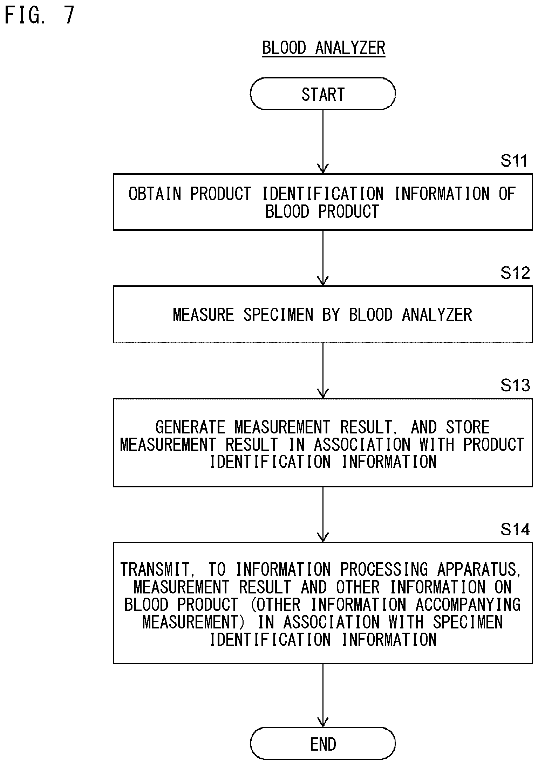

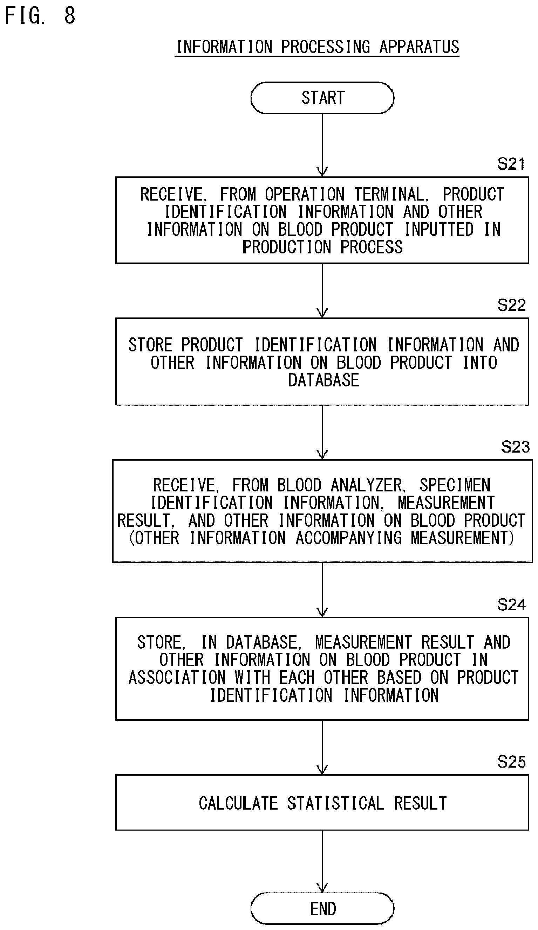

Next, processes performed by the blood analyzer 1 and the information processing apparatus 2 will be described with reference to the flowcharts shown in FIGS. 7 and 8.

FIG. 7 is a flowchart showing the process performed by the blood analyzer 1.

In step S11, the controller 121 of the blood analyzer 1 obtains product identification information of a blood product. As described above, a barcode label using the product identification information is attached as specimen identification information to a specimen container supplied to the blood analyzer 1. The controller 121 instructs the measurement controller 111 to read the specimen identification information from the barcode label on the specimen container, and the measurement controller 111 drives the reader 113.

In step S12, the controller 121 instructs the measurement controller 111 to measure the specimen with the blood analyzer 1. Specifically, the measurement controller 111, having received the instruction, drives the sample preparation unit 114 such that the specimen is aspirated from the specimen container and a measurement sample is prepared from the specimen. Then, the measurement controller 111 drives the detector 115 to measure the measurement sample. The signal processing circuit 116 generates measurement data on the basis of detection signals outputted from the components of the detector 115.

In step S13, the controller 121 generates a specimen measurement result on the basis of the measurement data generated by the signal processing circuit 116, and stores the generated measurement result into the storage unit 122 in association with the specimen identification information (product identification information) obtained in step S11. In addition, the controller 121 stores, in the storage unit 122, the measurement date/time of the specimen in association with the specimen identification information.

In step S14, the controller 121 transmits, to the information processing apparatus 2, the measurement result and the other information on the blood product other than the measurement result (other information accompanying measurement) in association with the specimen identification information. As described above, the other information accompanying measurement in this case includes the measurement date/time. The process in step S14 may be automatically executed without an instruction of the operator each time the process in step S13 is completed, or may be executed in response to the operator operating the input unit 124.

FIG. 8 is a flowchart showing the process performed by the information processing apparatus 2.

In step S21, the controller 201 of the information processing apparatus 2 receives, from the operation terminal, the product identification information and the information on the blood product other than the measurement result that have been inputted in the production process. In step S22, the controller 201 stores, in the database 2a of the storage unit 202, the product identification information and the other information on the blood product that have been received in step S21.

In step S23, the controller 201 receives the specimen identification information, the measurement result, and the other information on the blood product (other information accompanying measurement) that have been transmitted from the blood analyzer 1 in step S14 shown in FIG. 7. In step S24, the controller 201 automatically stores, in the database 2a, the measurement result and the other information on the blood product (other information accompanying measurement) received in step S23 so that the measurement result and the other information are associated with each other based on the product identification information. Thus, as shown in FIG. 4, the measurement result and the other information on the blood product stored in steps S22 and S24 are automatically associated with each other based on the product identification information. As described above, since the product identification information is used as the specimen identification information, the measurement result and the other information on the blood product can be associated with each other based on the product identification information. The word "automatically" means without an intervening operation by the operator. The process in step S24 may be executed through an operation by the operator.

In step S25, the controller 201 aggregates the measurement results of a plurality of specimens to calculate statistical results. The process in step S25 can be omitted.

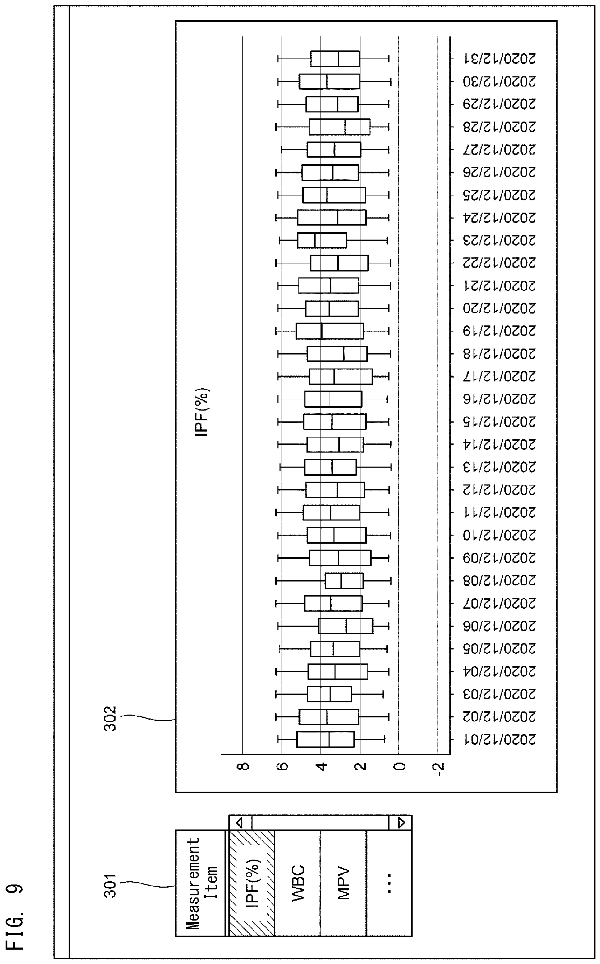

Next, examples of screens displayed on the display 203 of the information processing apparatus 2 will be described with reference to FIGS. 9 to 12. Each of the screens shown in FIGS. 9 to 12 is displayed on the display 203 by the controller 201 in response to a display instruction that is inputted by the operator operating the input unit 204.

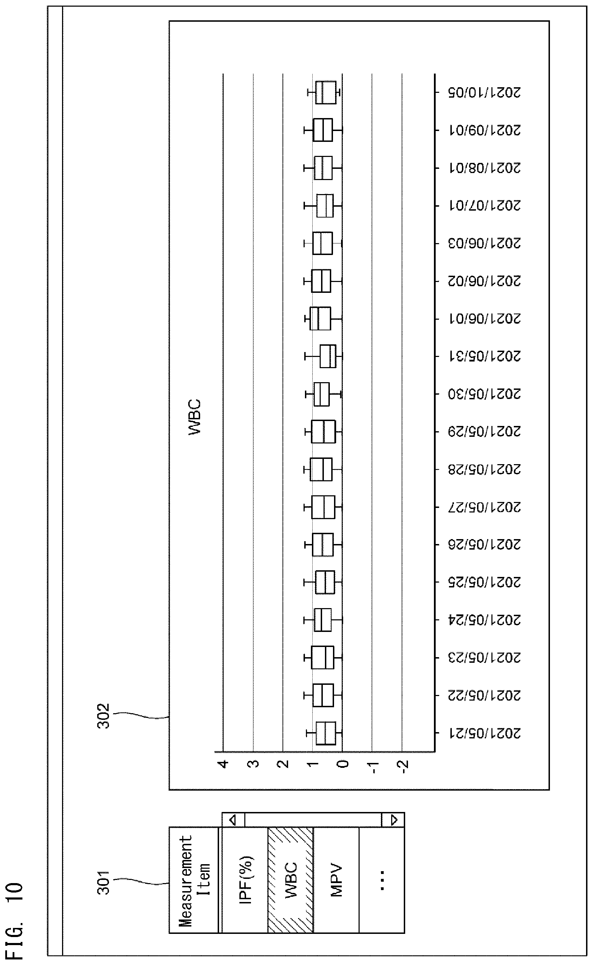

FIGS. 9, 10 each schematically show a screen displaying, in time series, statistical results obtained by aggregating the measurement results.

The operator selects a measurement item displayed in a measurement item selection area 301. Then, in a graph area 302, statistical results obtained by aggregating the measurement results of the blood product measured with respect to the selected measurement item over a period corresponding to predetermined measurement dates/times, are displayed in time series.

In the example shown in FIG. 9, a measurement item "IPF(%)" (immature platelet fraction) is selected out of the measurement results of the platelet product, and a box plot representing aggregation results of IPF(%) is displayed in the graph area 302. In the example shown in FIG. 10, a measurement item "WBC" (residual white blood cell count) is selected out of the measurement results of the platelet product, and a box plot representing aggregation results of WBC is displayed in the graph area 302. By referring to the screen as shown in FIGS. 9, 10, the operator can grasp not only the measurement results of the individual specimen (blood product) but also data distribution of the measurement results.

The screen shown in FIGS. 9, 10 may be provided with an operation part (e.g., a pull-down menu) for receiving the other information on the blood product (e.g., blood center, blood collection facility, type of collected blood, blood type, product type, factory ID, production date/time, measurement date/time, etc.). In this case, statistical results are generated by aggregating the measurement results corresponding to information designated on the operation part by the operator, and the generated statistical results are displayed in the graph area 302. This allows the operator to perform comparison with respect to different periods, factories, and blood collection facilities.

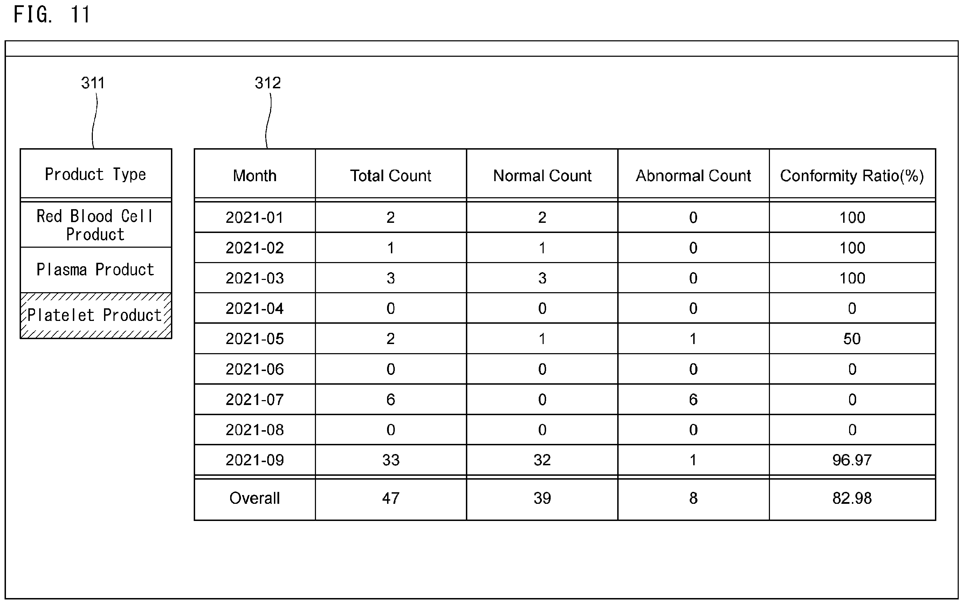

FIG. 11 schematically shows a screen displaying, for each period, the statistical results obtained by aggregating the measurement results.

When the operator selects a product type displayed in a product type selection area 311, statistical results obtained by aggregating the measurement results of a blood product corresponding to the product type selected in the product type selection area 311, are displayed for each period in a list area 312.

In the example shown in FIG. 11, "platelet product" is selected from among the product types, and the result of aggregation for each month to which the measurement dates/times belong, with respect to the platelet product, is displayed in the list area 312. The list area 312 includes, as items, month, total count (the total number of specimens), normal count (the number of normal specimens), abnormal count (the number of abnormal specimens), and conformity ratio (%). The conformity ratio is a value obtained by dividing the number of normal specimens by the total number of specimens. By referring to the screen as shown in FIG. 11, the operator can grasp monthly trends of a specific product type.

The screen shown in FIG. 11 may also be provided with an operation part for receiving the other information on the blood product (e.g., blood center, blood collection facility, type of collected blood, blood type, factory ID, production date/time, measurement date/time, etc.).

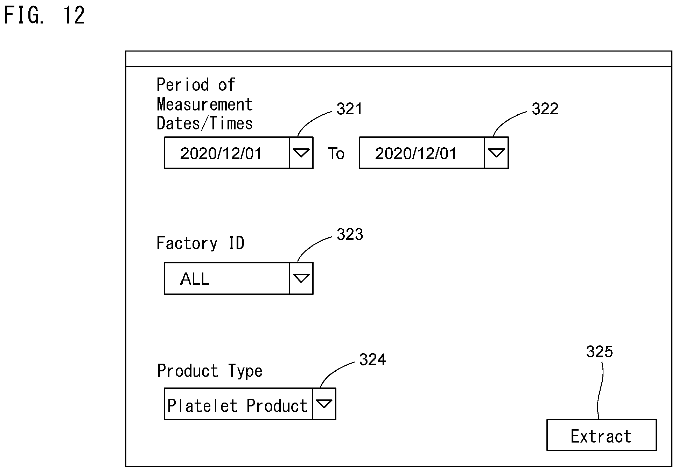

FIG. 12 schematically shows a screen for inputting an instruction to extract and display measurement results.

The operator operates pull-down menus 321 to 324 to select conditions for extracting measurement results. When the operator operates an extraction button 325, measurement results are extracted based on the conditions selected via the pull-down menus 321 to 324, and statistical results of the extracted measurement results are displayed on the display 203. In the example shown in FIG. 12, the pull-down menus 321, 322 for selecting a period of measurement dates/times, the pull-down menu 323 for selecting a factory ID, and the pull-down menu 324 for selecting a product type are arranged on the screen.

The screen shown in FIG. 12 may be provided with an operation part for selecting the other information on the blood product (e.g., blood center, blood collection facility, type of collected blood, blood type, production date/time, etc.).

The operator can appropriately combine the conditions on the screen shown in FIG. 12, and can cause the display 203 to display the statistical results calculated from the measurement results of the blood product that meet the conditions. This allows the operator to evaluate the test results of the blood product from a plurality of viewpoints.

### <Effects of management method, information processing apparatus, and information management system according to Embodiment 1>

The controller 201 of the information processing apparatus 2 obtains: a measurement result of a specimen, collected from a blood product, which is measured by the blood analyzer 1 with product identification information of the blood product being used as specimen identification information of the specimen; and the specimen identification information (step S23 in FIG. 8). In the database 2a in which information on the blood product is stored in association with the product identification information, the controller 201 associates the measurement result with other information on the blood product other than the measurement result, on the basis of the product identification information used as the specimen identification information (step S24 in FIG. 8).

According to the above process, by using the product identification information of the blood product as the specimen identification information of the specimen collected from the blood product, the specimen identification information of the specimen and the measurement result of the specimen measured by the blood analyzer 1 are obtained, and the obtained measurement result and the other information on the blood product can be associated with the product identification information. This enables unitary management of the measurement result of the specimen measured by the blood analyzer 1 and the other information on the blood product. The phrase "unitary management" does not necessarily mean that the measurement result and the other information on the blood product are stored in a single information processing apparatus. Even when the measurement result and the other information on the blood product are separately stored in a plurality of information processing apparatuses, these pieces of information may be associated with each other based on the product identification information.

The product identification information is identification information assigned to a blood bag that contains blood collected from a donor. According to this configuration, unitary management of pieces of information is performed based on the product identification information assigned to the blood bag in the blood collection facility, and therefore, the pieces of information can be unitarily managed retroactively to the time of blood collection.

The product identification information includes a number obtained by encoding at least a part of the other information on the blood product other than the measurement result. The at least a part of the other information on the blood product includes, for example, blood center, blood collection facility, type of collected blood, and blood type. Such product identification information attached to a bag or the like of the blood product prevents mix-up of the blood product, and the information associated with the blood product can be reliably obtained.

The other information on the blood product includes information regarding at least one of: the property of the blood product; the blood product producing process, and the blood product measurement process. The property of the blood product includes the blood type, the product type, and the like, for example. The blood product producing process includes: the blood collection process in the blood collection facility shown in FIG. 1; and the production process of actually producing the blood product shown in FIG. 1 and the like. The blood product measurement process includes the test process shown in FIG. 1 and the like. This configuration enables unitary management of the measurement result, the property of the blood product, and the information on each process.

The controller 201 of the information processing apparatus 2 selects at least one blood product from among a plurality of blood products registered in the database 2a on the basis of the other information on the blood product, and further generates information by aggregating the measurement results of specimens collected from the at least one blood product selected (step S25 in FIG. 8), as shown in FIGS. 9 to 11. This process enables narrowing to one or a plurality of blood products to be evaluated, and collective confirmation of the measurement results of the blood products.

### <Modification 1 of Embodiment 1>

In the test process of Embodiment 1, the operator may input a measurement order into the blood analyzer 1.

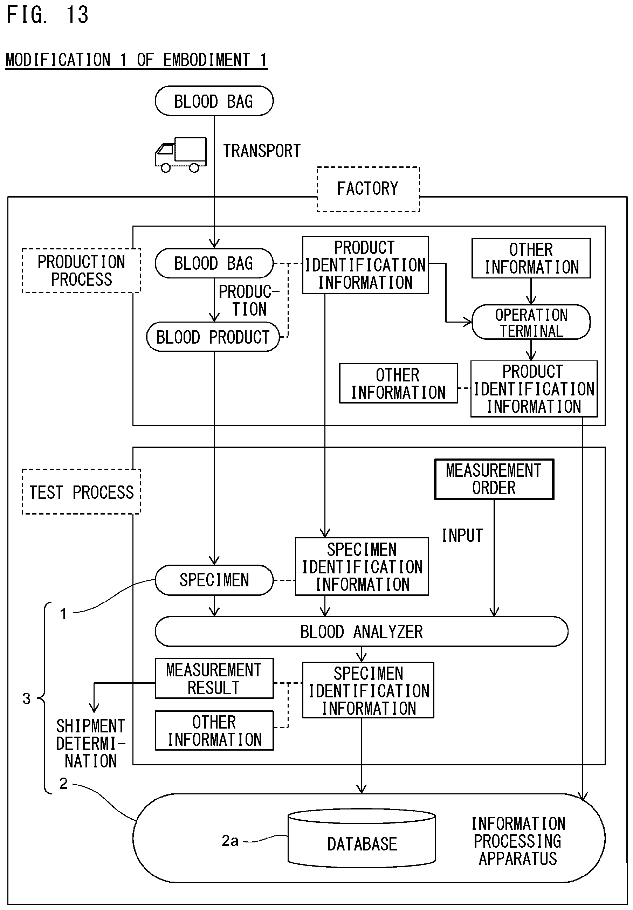

FIG. 13 schematically shows a series of processes including blood collection, production of blood products, and testing, according to Modification 1 of Embodiment 1.

In FIG. 13, illustration of the blood collection process is omitted for convenience. In FIG. 13, in contrast to FIG. 1, the operator inputs a measurement order into the blood analyzer 1 on the basis of the other information on the blood product, specifically, the product type of the blood product produced in the production process.

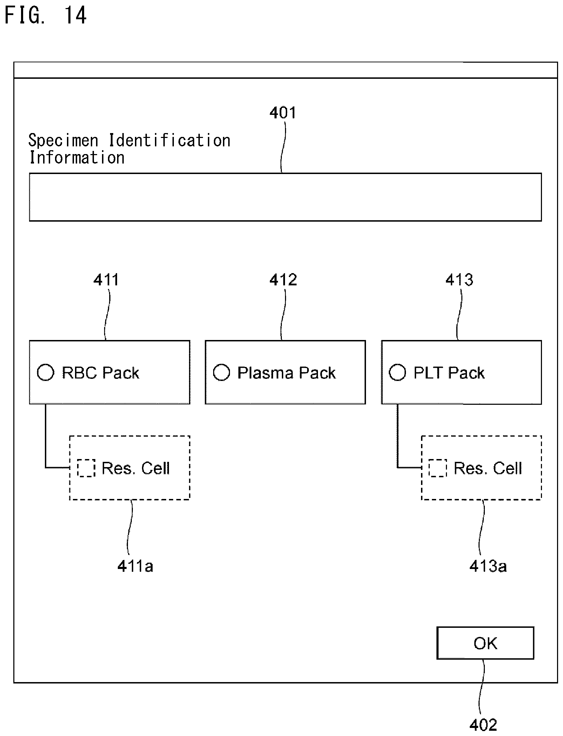

FIG. 14 schematically shows a screen, for inputting a measurement order, displayed on the display 123 of the blood analyzer 1 (see FIG. 5). The screen shown in FIG. 14 is displayed on the display 123 by the controller 121 in response to a display instruction that is inputted by the operator operating the input unit 124.

The operator operates the input unit 124 (see FIG. 5) and the reader 113 (see FIG. 5) to input specimen identification information indicated by a barcode label adhered to a specimen container into a text box 401.

When selecting measurement orders of a red blood cell product, a plasma product, and a platelet product, the operator selects radio buttons 411 to 413, respectively. When the measurement order of the red blood cell product is selected, the blood analyzer 1 measures a red blood cell count. When the measurement order of the plasma product is selected, the blood analyzer 1 measures a residual white blood cell count and a residual red blood cell count. When the measurement order of the platelet product is selected, the blood analyzer 1 measures a platelet count. When the radio buttons 411, 413 are selected, check boxes 411a, 413a are enabled, respectively. When the check box 411a is selected, a residual white blood cell count is further measured in measurement of the red blood cell product. When the check box 413a is selected, a residual white blood cell count and a residual red blood cell count are further measured in measurement of the platelet product.

When an OK button 402 is operated by the operator, the controller 121 (see FIG. 5) stores, in the storage unit 122, the measurement order based on the selection states of the radio buttons 411, 412, 413 and the check boxes 411a, 413a, in association with the specimen identification information inputted to the text box 401.

Thereafter, the specimen container is supplied to the blood analyzer 1, and the specimen identification information is read from the specimen container by the reader 113. Then, the measurement controller 111 drives the sample preparation unit 114 so as to prepare a measurement sample on the basis of the measurement order corresponding to the read specimen identification information, and drives the detector 115 so as to measure the measurement sample.

According to this modification, since the measurement order can be inputted through the screen shown in FIG. 14, measurement of the specimen collected from the blood product can be smoothly started.

### <Modification 2 of Embodiment 1>

In Modification 1 of Embodiment 1, the measurement order is inputted through the screen shown in FIG. 14. However, the information processing apparatus 2 may determine a measurement order on the basis of the product type, and the blood analyzer 1 may obtain the measurement order from the information processing apparatus 2.

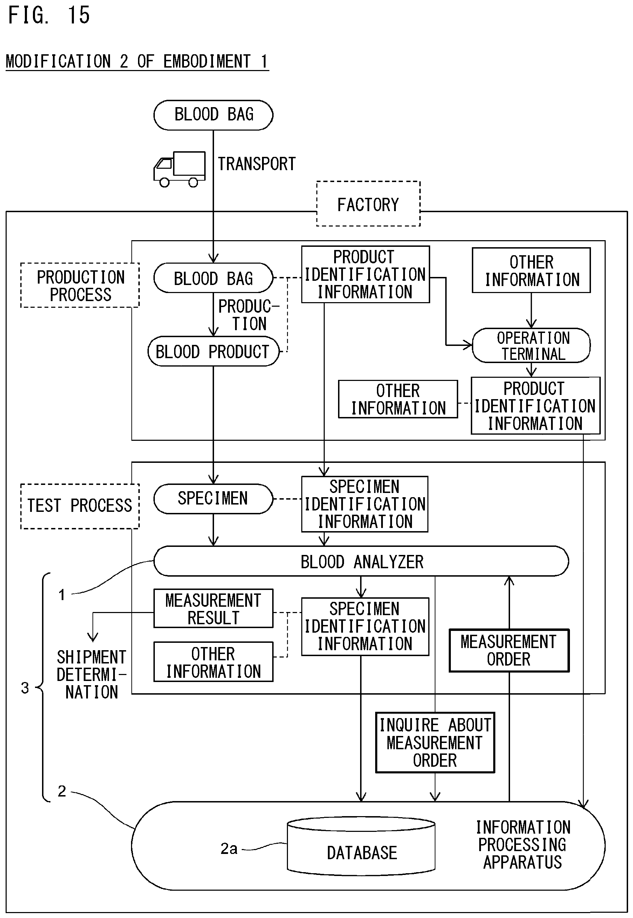

FIG. 15 schematically shows a series of processes including blood collection, production of blood products, and testing, according to Modification 2 of Embodiment 1.

In FIG. 15, in contrast to FIG. 13, the operator does not input a measurement order, and a measurement order is transmitted from the information processing apparatus 2 to the blood analyzer 1 in response to an inquiry of the blood analyzer 1.

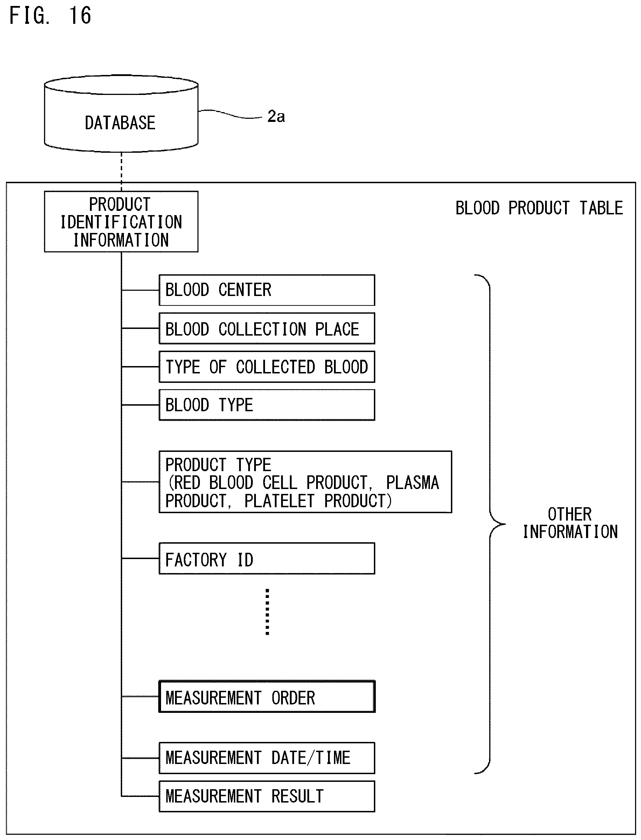

The controller 201 of the information processing apparatus 2 generates a measurement order on the basis of the other information on the blood product transmitted in the production process. Specifically, the controller 201 generates the same measurement order as that in the example shown in FIG. 14 on the basis of the product type. However, regarding information as to whether or not residual blood cells should be measured (information corresponding to the check box 411a, 413a in FIG. 14), the operator sets in advance whether or not to execute the measurement, on the information processing apparatus 2. In this modification, as shown in FIG. 16, the controller 201 stores the generated measurement order in association with the product identification information in the database 2a.

When a specimen container is supplied to the blood analyzer 1, and specimen identification information is read from the specimen container by the reader 113, the controller 121 of the blood analyzer 1 performs an inquiry about the measurement order to the information processing apparatus 2 on the basis of the specimen identification information. The controller 201 of the information processing apparatus 2 replaces the specimen identification information included in the received inquiry with product identification information, and reads the measurement order from the database 2a. The controller 201 transmits, to the blood analyzer 1, the read measurement order in association with the specimen identification information. The controller 121 of the blood analyzer 1 stores the received measurement order in association with the specimen identification information, and starts measurement based on the measurement order.

According to the modification, the measurement order is automatically generated according to the product type, and stored in the database 2a. Then, the measurement order is transmitted from the information processing apparatus 2 to the blood analyzer 1 in response to the inquiry about the measurement order from the blood analyzer 1. Thus, measurement of the specimen collected from the blood product can be smoothly started while omitting the labor of the operator.

### <Modification 3 of Embodiment 1>

In Modification 1 of Embodiment 1, the product identification information is used as the specimen identification information. However, in the test process, specimen identification information for identifying a specimen may be given irrespective of product identification information.

In this case, upon completion of step S13 in FIG. 7, in step S 14, the blood analyzer 1 automatically transmits, to the information processing apparatus 2, the measurement result and the other information on the blood product (other information accompanying measurement) in association with the product identification information obtained in step S11. The information processing apparatus 2 receives the product identification information, the measurement result, and the other information on the blood product (other information accompanying measurement) from the blood analyzer 1 in step S23 in FIG. 8, and automatically stores, in the database 2a, the measurement result and the other information on the blood product in association with each other based on the product identification information in step S24.

### <Effects of management method, information processing apparatus, and information management system according to Modification 3 of Embodiment 1>

According to this modification, the measurement result of the blood product measured by the blood analyzer 1 and the other information on the blood product can be unitarily managed in association with each other in the database 2a. Moreover, since transmission of the measurement result and the product identification information from the blood analyzer 1 and association of the measurement result of the blood product with the other information on the blood product in the database 2a, are automatically performed, unitary management of these pieces of information can be easily and appropriately performed.

### <Embodiment 2>

In Embodiment 1, the product identification information is used as it is, as the specimen identification information. In Embodiment 2, however, information obtained by adding additional information to product identification information is used as specimen identification information. Embodiment 1 and Embodiment 2 have a commonality in that the product identification information is used as the specimen identification information.

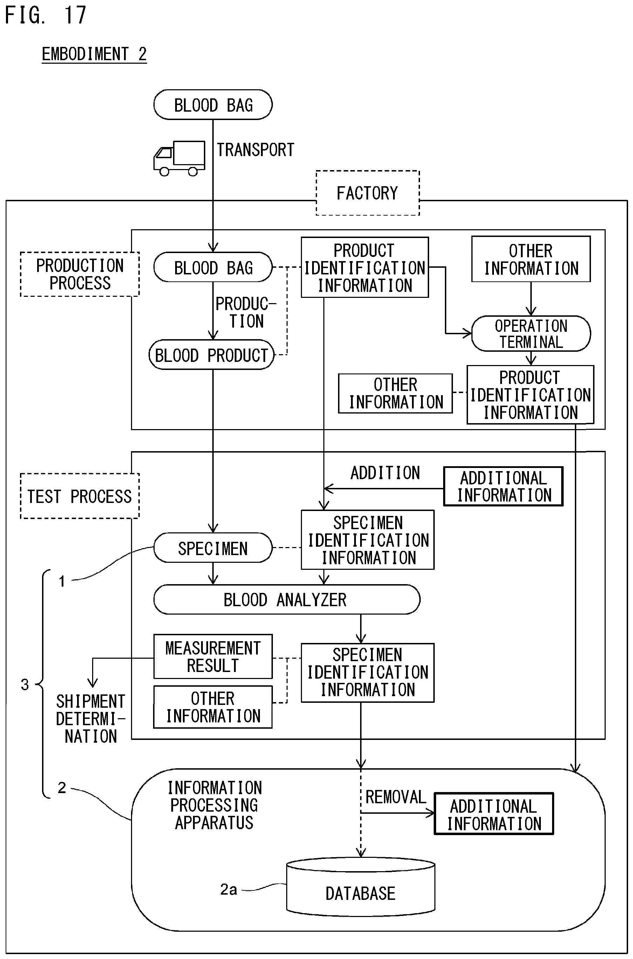

FIG. 17 schematically shows a series of processes including blood collection, production of blood products, and testing, according to Embodiment 2.

In FIG. 17, in contrast to FIG. 1, additional information is added to the product identification information before the specimen identification information is inputted to the blood analyzer 1, and after the measurement result is generated by the blood analyzer 1, the additional information is removed from the specimen identification information before storage of the specimen identification information into the database 2a.

For example, additional information composed of a predetermined number or character string is added to the end of the product identification information, and information composed of the product identification information and the additional information is used as specimen identification information. In this case, on a barcode label to be adhered to a specimen container, a barcode indicating the newly set specimen identification information is printed. Upon receiving the specimen identification information, the measurement result, and the other information on the blood product (other information accompanying measurement) from the blood analyzer 1, the controller 201 of the information processing apparatus 2 removes the additional information from the specimen identification information, and stores, in the database 2a, the measurement result and the other information on the blood product in association with the product identification information after the removal.

Addition of the additional information to the product identification information and removal of the additional information from the specimen identification information may be performed by the controller 121 of the blood analyzer 1.

### <Modification of Embodiment 2>

In Embodiment 2, the product identification information to which the additional information is added is used as the specimen identification information. Alternatively, a number or a character string obtained by converting product identification information according to a predetermined rule may be used as specimen identification information. In this case, after generation of the measurement result, the specimen identification information is restored to the product identification information according to a predetermined rule. This modification and Embodiments 1 and 2 have a commonality in that the product identification information is used as the specimen identification information.

For example, upon receiving the product identification information, the blood analyzer 1 adds a predetermined integer (e.g., 1) to each of the digits of the product identification information, thereby generating specimen identification information composed of digits corresponding to the lowest digits of the arithmetic results. Upon receiving the measurement result and the like, the controller 201 of the information processing apparatus 2 subtracts the predetermined integer (e.g., 1) from each of the digits of the specimen identification information to obtain the original product identification information. Specifically, assuming that the product identification information is "37-2134-0639", the specimen identification information becomes "48-3245-1740" by adding "1" to each digit. In order to obtain the original product identification information from the specimen identification information, "1" is subtracted from each digit of the specimen identification information.

### <Embodiment 3>

In Embodiment 1, one blood analyzer 1 is installed in one factory. In embodiment 3, a plurality of blood analyzers 1 are installed in one factory.

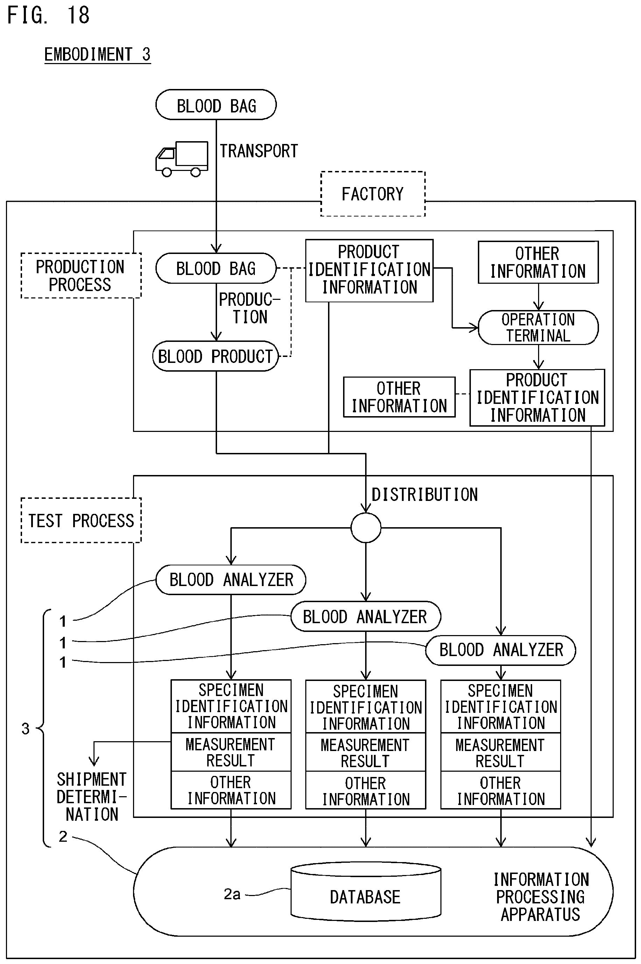

FIG. 18 schematically shows a series of processes including blood collection, production of blood products, and testing, according to Embodiment 3.

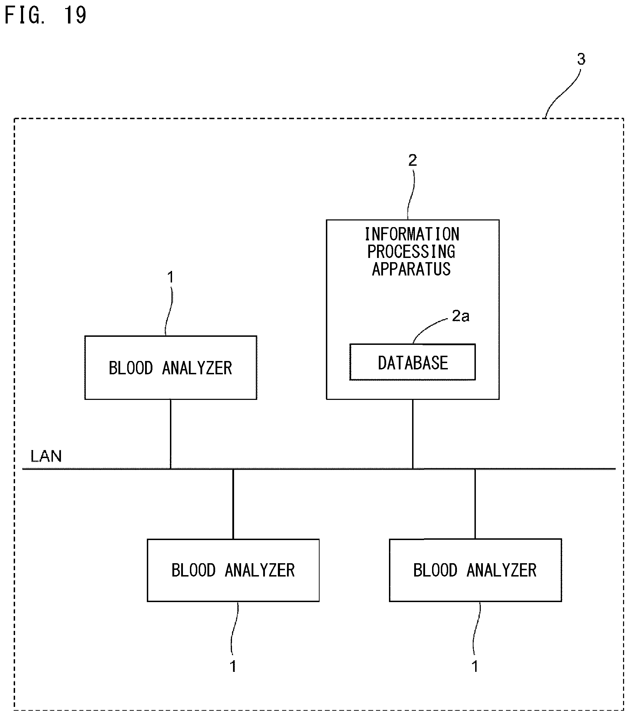

In FIG. 18, in contrast to FIG. 1, three blood analyzers 1 are installed in a factory. The three blood analyzers 1 and one information processing apparatus 2 constitute an information management system 3. As shown in FIG. 19, the three blood analyzers 1 and the one information processing apparatus 2 are connected to a LAN in the factory. This connection allows the three blood analyzers 1 and the one information processing apparatus 2 to communicate with each other.

In the production process, as in Embodiment 1, product identification information of all blood products and other information on the blood products other than measurement results, are stored in the database 2a. The blood products produced in the production process are distributed to the three blood analyzers 1 according to a predetermined rule. The predetermined rule is, for example, a rule for distributing the blood products such that the three blood analyzers 1 have roughly the same number of specimens to be processed, or a rule for distributing the blood products to the three blood analyzers 1 according to three time bands (morning, afternoon, and night). Each of the distributed blood products is measured by the corresponding blood analyzer 1, and a measurement result and other information on the blood product other than the measurement result are stored in the database 2a in association with specimen identification information.

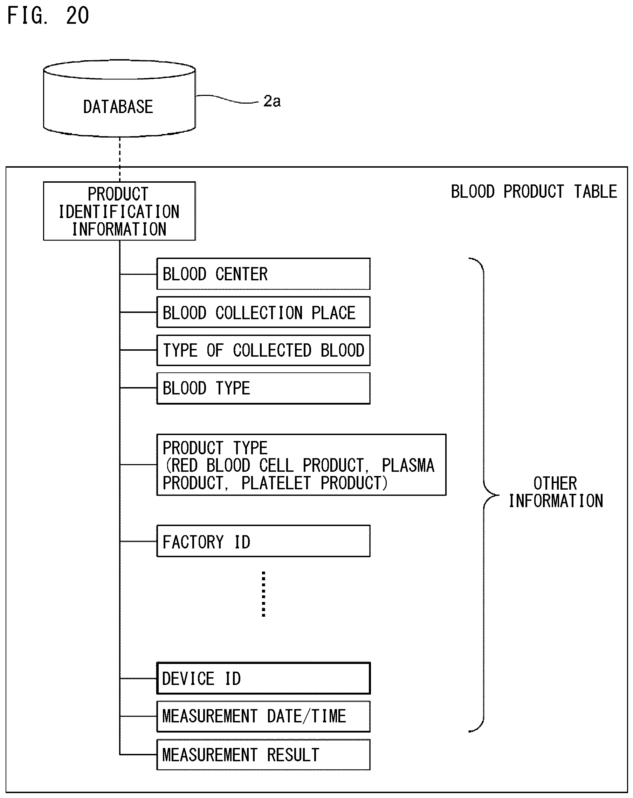

FIG. 20 schematically shows items stored in the database 2a.

In contrast to FIG. 4, the database 2a of Embodiment 3 includes device IDs capable of individually identifying the blood analyzers 1. In FIG. 18, the other information on the blood product (other information accompanying measurement) transmitted from each blood analyzer 1 to the information processing apparatus 2 includes the device ID corresponding to the blood analyzer 1. Thus, when referring to the measurement result displayed on the information processing apparatus 2, the operator can grasp which blood analyzer 1 has generated the measurement result.

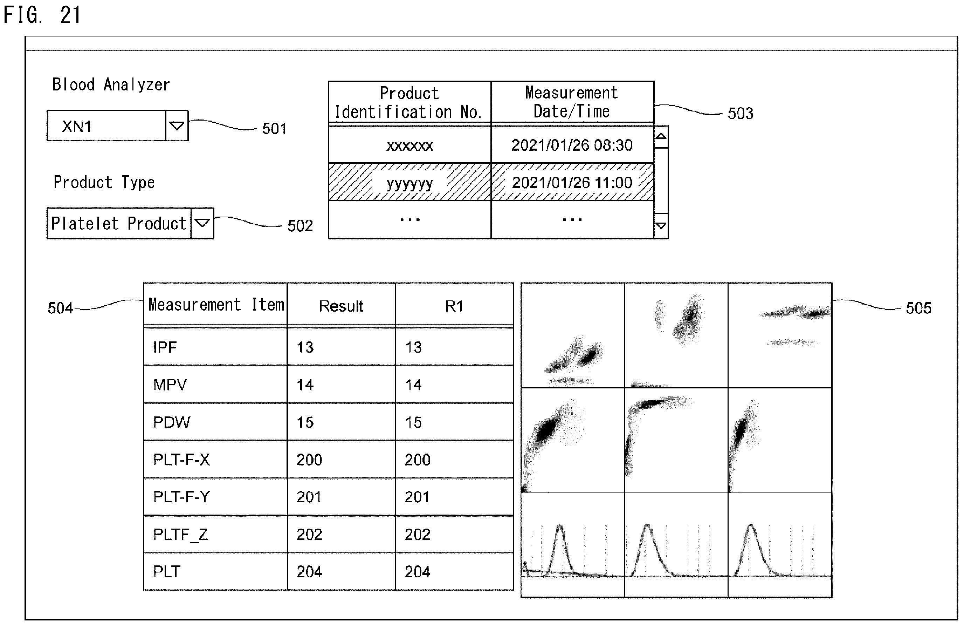

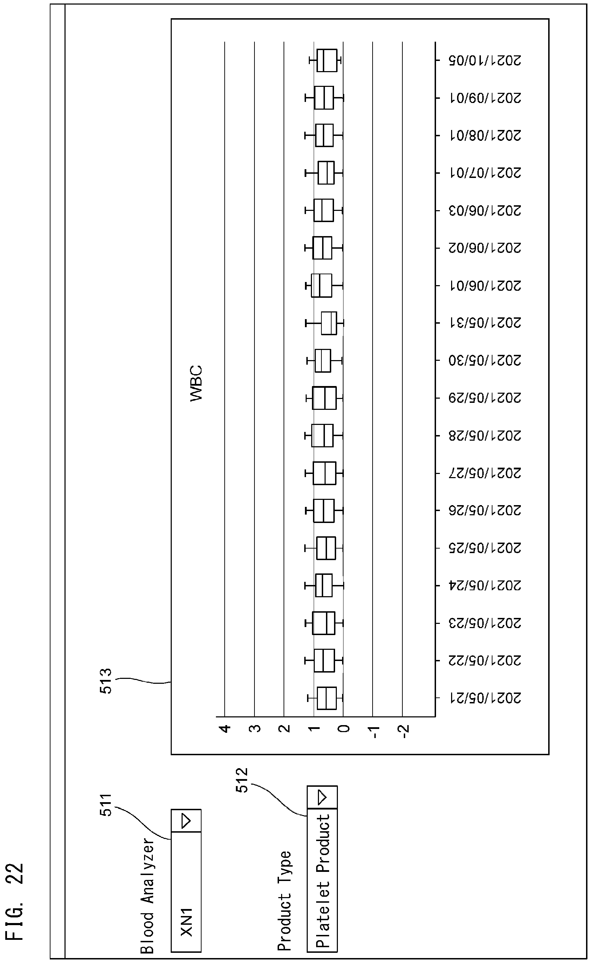

Next, with reference to FIGS. 21, 22, examples of screens displayed on the display 203 of the information processing apparatus 2 will be described. Each of the screens shown in FIGS. 21, 22 is displayed on the display 203 by the controller 201 in response to a display instruction that is inputted by the operator operating the input unit 204.

FIG. 21 schematically shows a screen displaying individual measurement results.

When the operator selects a device ID by operating a pull-down menu 501 and selects a product type by operating a pull-down menu 502, a list of corresponding blood products is displayed in a blood product selection area 503. When the operator selects a blood product by operating the blood product selection area 503, measurement results of the selected blood product are displayed for each measurement item in a list area 504, and graphs related to the measurement results are displayed in a graph area 505. This allows the operator to confirm detailed measurement results for each blood product.

The screen shown in FIG. 21 may be provided with an operation part for receiving the other information on the blood product. Thus, the measurement results to be displayed can be further narrowed down.

FIG. 22 schematically shows a screen displaying, in time series, statistical results obtained by aggregating the measurement results.

When the operator selects a device ID by operating a pull-down menu 511 and selects a product type by operating a pull-down menu 512, the measurement results of the selected product type, out of the measurement results obtained by the blood analyzer 1 of the selected device ID, are displayed in a graph area 513. In the graph area 513, statistical results obtained by aggregating the measurement results obtained over a period corresponding to predetermined measurement dates/times are displayed in time series. By referring to the screen shown in FIG. 22, the operator can grasp data distribution of the measurement results obtained by the selected blood analyzer 1.

The screen shown in FIG. 22 may be provided with an operation part for receiving the other information on the blood product. Thus, the measurement results to be displayed can be further narrowed down.

### <Effects of management method, information processing apparatus, and information management system according to Embodiment 3>

As shown in FIG. 19, the information management system 3 includes the plurality of blood analyzers 1, and as shown in FIG. 20, information on the blood product measurement process includes a device ID for specifying a blood analyzer 1 that has performed specimen measurement. According to this configuration, in a facility where a plurality of blood analyzers 1 are installed, information utilization can be performed taking into account a difference between the blood analyzers 1.

### <Embodiment 4>

In Embodiment 4, blood products are grouped according to a predetermined condition, and information on the grouped blood products is used as quality control information. A series of processes including blood collection, production of blood products, and testing according to Embodiment 4 are identical to those shown in FIG. 1.

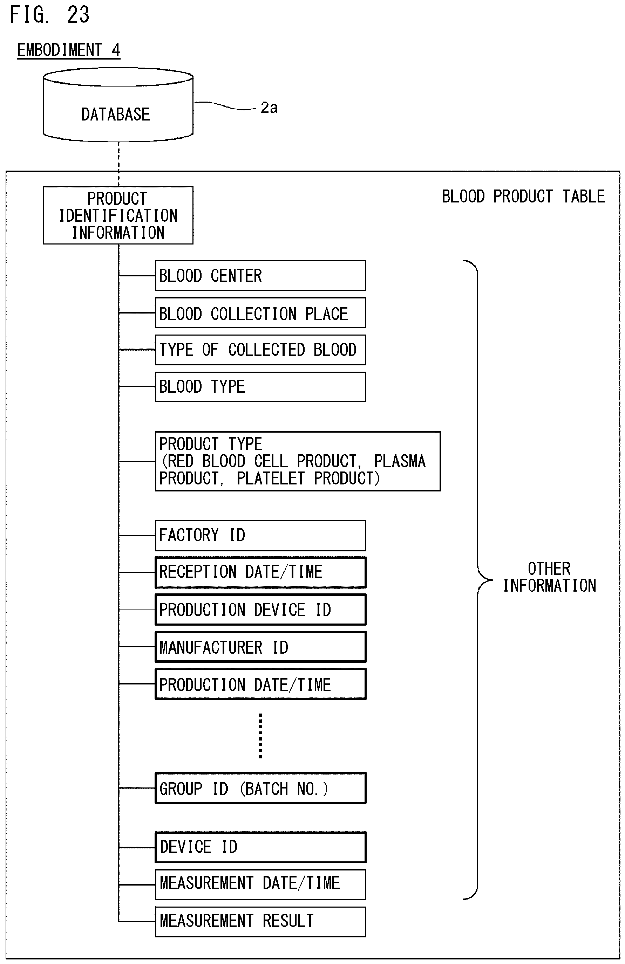

FIG. 23 schematically shows items stored in the database 2a, according to Embodiment 4.

In contrast to FIG. 4, the database 2a according to Embodiment 4 includes items such as reception date/time, production device ID, manufacturer ID, production date/time, device ID, and group ID on the blood product table. As described above, the reception date/time is when the factory received the blood bag. The production device ID includes a lot number of a white blood cell removal filter used in the production process, identification information of a centrifuge used in the production process, identification information of a blood component collection apparatus used in the blood component collection, and the like. The manufacturer ID is information for identifying an operator who produced the blood product. The production date/time is when the blood product was produced. The device ID is information for identifying the blood analyzer 1. The group ID is an ID for grouping product identification information registered in the database 2a.

When a group of product identification information having the same group ID is set, grouped blood products can be subjected to quality evaluation with reference to the measurement results of the group.

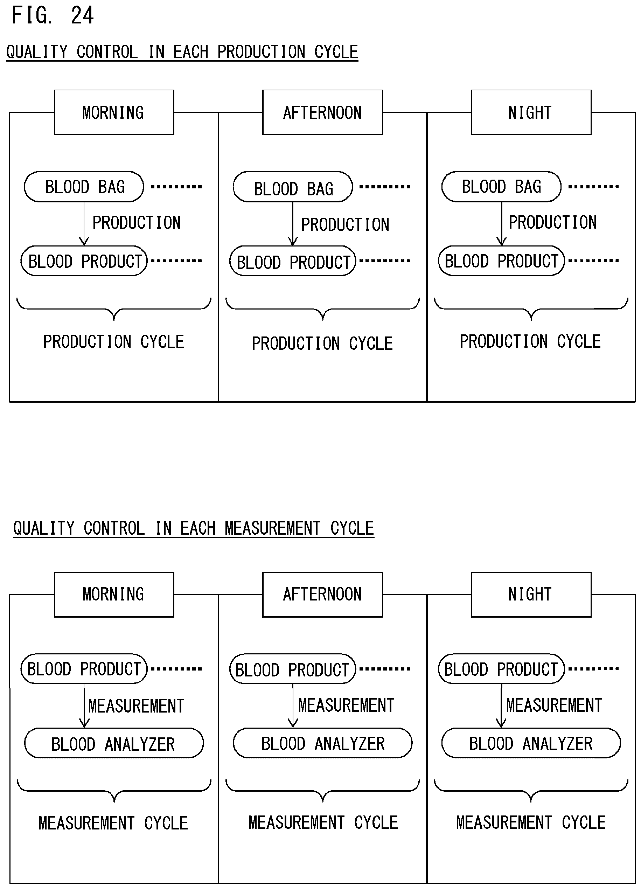

FIG. 24 schematically shows an example in which quality control is performed in each production cycle and in each measurement cycle.

As shown in the upper part of FIG. 24, generally, blood bags are collectively transported to the factory for each of three time bands, i.e., morning, afternoon, and night. In the production process, blood products are produced from the blood bags in each time band. One time band corresponds to one production cycle. In the same production cycle, the production process including, for example, the condition at which the blood products are produced (temperature, humidity, state of production facility, etc.) and the technique of the manufacturer involved in the production is assumed to be uniform. Therefore, grouping the blood products produced in one production cycle enables appropriate evaluation of the quality of the blood products for each group unit.

As shown in the lower part of FIG. 24, in the test process, generally, measurement of the blood products is performed in three separate time bands, i.e., morning, afternoon, and night. One time band corresponds to one measurement cycle. In the same measurement cycle, it is assumed that the state of the blood analyzer 1 used for measurement is uniform. Therefore, grouping the blood products measured in one measurement cycle enables appropriate evaluation of the quality of the blood products for each group unit.

Usually, blood products produced together in the same production cycle are measured in the same measurement cycle. A unit of products produced together in the same production cycle and measured together in the same test cycle is referred to as a "batch". For example, in a factory in which blood collected in blood collection facilities in each region arrives periodically (e.g., in the morning, noon, and evening), production and measurement of blood products are performed periodically (e.g., in the morning, afternoon, and night) with a unit of arrival being "1 batch". A batch consists of one or a plurality of blood products, and preferably, consists of a plurality of blood products. Measurement performed in the measurement cycle corresponding to 1 batch may be performed in one blood analyzer 1 or may be performed by a plurality of blood analyzers 1 in parallel.

The upper part of FIG. 24 indicates an example in which blood products whose "production date/time" matches "specific time band on that day" (one production cycle) are grouped, and the lower part of FIG. 24 indicates an example in which blood products whose "measurement date/time" matches "specific time band on that day" (one measurement cycle) are grouped. However, grouping for the purpose of quality control is not limited thereto.

For example, blood products whose "reception date/time" corresponds to "that day" may be grouped for each "blood collection facility", blood products whose "reception date/time" corresponds to "that day" may be grouped for each "manufacturer ID", blood products whose "reception date/time" corresponds to "that day" may be grouped for each "factory ID", or blood products whose "reception date/time" corresponds to "that day" may be grouped for each "production device ID".

Generation of groups as described above is automatically performed by the controller 201 of the information processing apparatus 2. In this case, when the current time has reached a set time (e.g., 12:00 or 17:00), the controller 201 stores a common number or character string as a group ID, and automatically generates a group of blood products. Alternatively, the controller 201 may automatically generate a group of blood products when the operator inputs a group generation instruction to the information processing apparatus 2.

Generation of groups as described above may be performed by the operator manually inputting a condition at any timing.

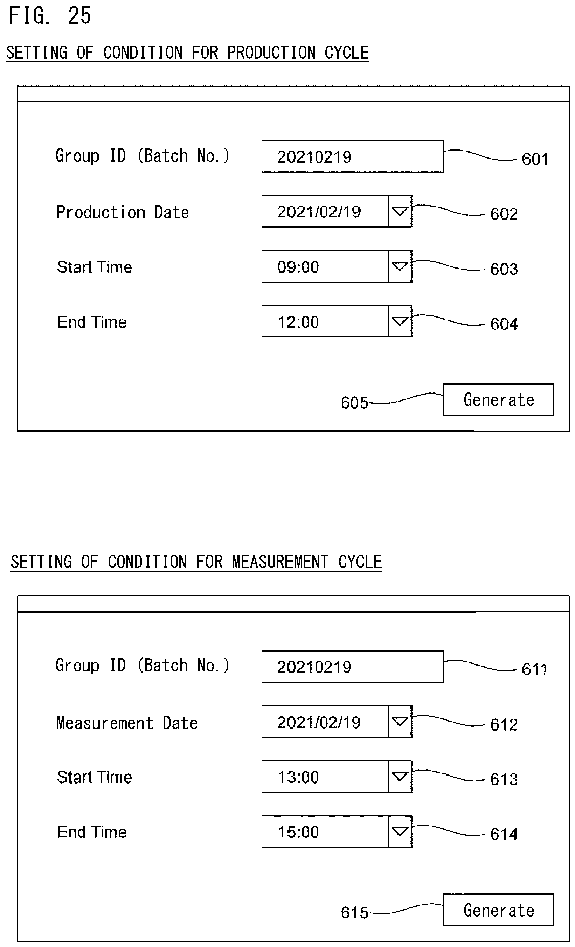

FIG. 25 schematically shows a screen for manually generating a group. The screen shown in FIG. 25 is displayed on the display 203 by the controller 201 in response to a display instruction that is inputted by the operator operating the input unit 204.

In FIG. 25, an upper screen is a screen for generating a group corresponding to a production cycle by inputting a range of a production date/time, and a lower screen is a screen for generating a group corresponding to a measurement cycle by inputting a range of a measurement date/time.

In the upper screen shown in FIG. 25, the operator inputs any group ID in a text box 601, and selects a production date, a start time, and an end time by operating pull-down menus 602 to 604, respectively. Then, when the operator operates a generation button 605, the controller 201 of the information processing apparatus 2 stores the group ID inputted to the text box 601, in the item for the group ID of the corresponding product identification information, thereby generating a group.

In the lower screen shown in FIG. 25, the operator inputs any group ID in a text box 611, and selects a measurement date, a start time, and an end time by operating pull-down menus 612 to 614, respectively. Then, when the operator operates a generation button 615, the controller 201 stores the group ID inputted to the text box 611, in the item for the group ID of the corresponding product identification information, thereby generating a group.

In FIG. 25, the product identification information is grouped according to the range of the production date/time and the range of the measurement date/time. However, the product identification information may be grouped according to information on the blood products other than the production date/time and the measurement date/time, by using the same screen as that shown in FIG. 25.

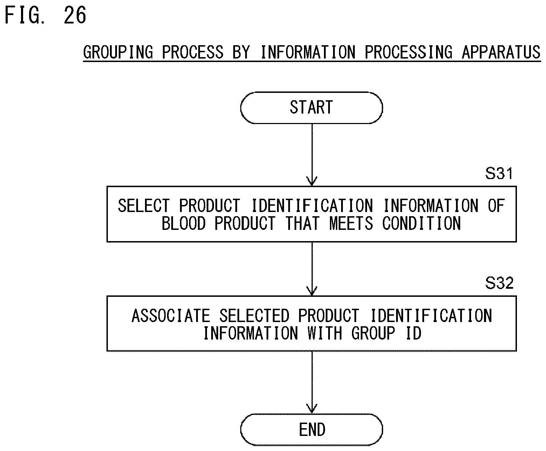

FIG. 26 is a flowchart showing a grouping process by the information processing apparatus 2.

In step S31, the controller 201 of the information processing apparatus 2 selects product identification information of blood products that match the condition, at a predetermined timing. As described above, the predetermined timing is, for example, when the current time has reached the set time, when the operator inputs a group generation instruction, or when the generation button 605 or 615 is operated on the screen as shown in FIG. 25. In step S32, the controller 201 associates the product identification information selected in step S31 with a group ID. That is, the controller 201 stores a common number or character string in the item for the group ID corresponding to the selected product identification information.

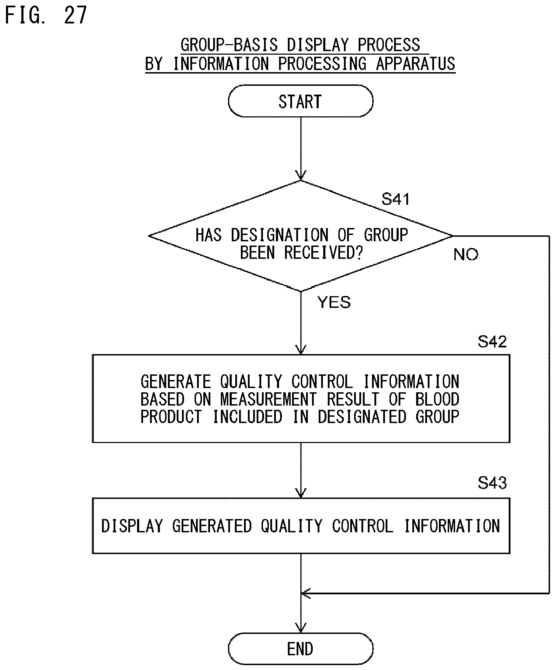

FIG. 27 is a flowchart showing a display process for each group by the information processing apparatus 2.

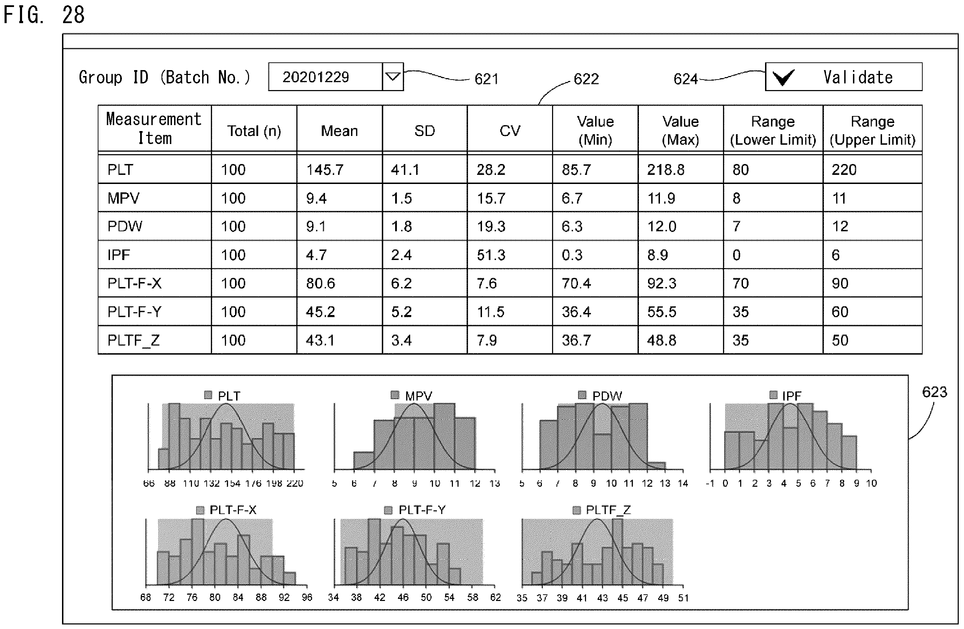

In step S41, the controller 201 determines whether or not designation of a group has been received. Designation of a group is executed by an operation of the operator to a pull-down menu described later with reference to FIGS. 28 to 31, for example. In step S42, the controller 201 generates quality control information based on the measurement results of blood products included in the group designated in step S41. In step S43, the controller 201 displays the quality control information generated in step S42 on the display 203 as described later with reference to FIGS. 28 to 31.

Next, examples of screens displayed on the display 203 of the information processing apparatus 2 will be described with reference to FIGS. 28 to 31. Each of the screens shown in FIGS. 28 to 31 is displayed on the display 203 by the controller 201 in response to a display instruction that is inputted by the operator operating the input unit 204.

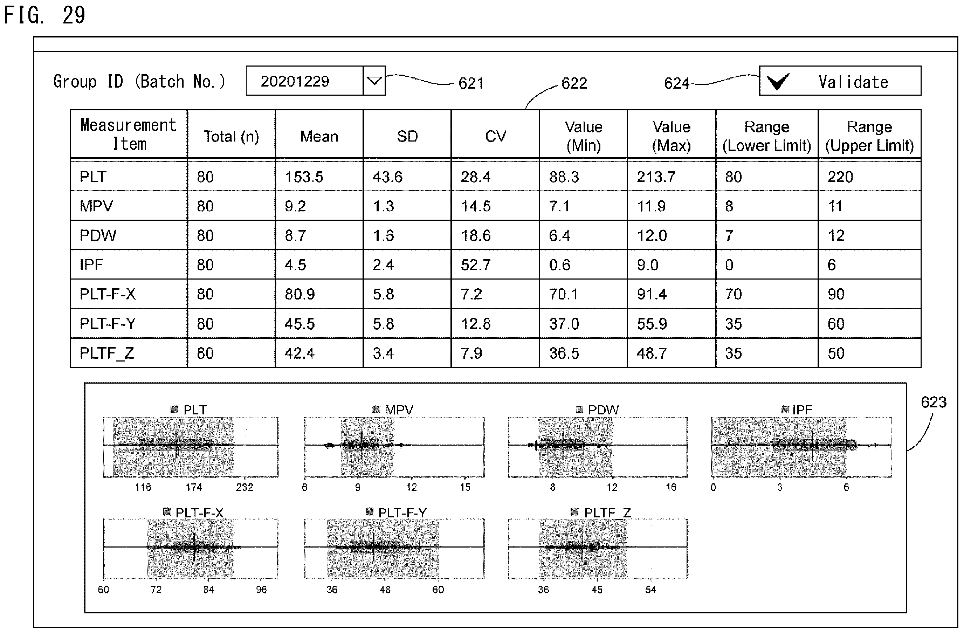

FIGS. 28, 29 each schematically show a screen displaying quality control information based on measurement results of blood products included in a group.

When the operator selects a group ID (batch No.) by operating a pull-down menu 621, statistical results obtained by aggregating the measurement results are displayed for each measurement item in a list area 622, and graphs indicating distributions of the values of the respective measurement items are displayed in a graph area 623. In this case, the list area 622 and the graph area 623 become the quality control information.

In the example shown in FIG. 28, the statistical results of grouped platelet products are displayed in the list area 622, and histograms indicating the values of the measurement items of the grouped platelet products are displayed in the graph area 623. In the example shown in FIG. 29, the statistical results of the grouped platelet products are displayed in the list area 622, and box plots indicating the values of the measurement items of the grouped platelet products are displayed in the graph area 623. By referring to the screens shown in FIGS. 28, 29, the operator can confirm the quality control information based on the measurement results of the blood products included in the designated group.

When the operator refers to the screens shown in FIGS. 28, 29 and determines that the displayed quality control information has no problem, the operator operates a validation button 624. Thus, the blood products included in this group are set to the ready-for-shipment states. The validation button 624 disposed on the screen displaying the quality control information allows the operator to confirm the quality control information and smoothly set the blood products to the ready-for-shipment states.

Since the blood products are produced from blood collected from healthy individuals, the operator can also confirm the states of reagents used in the blood analyzer 1 and the state of the blood analyzer 1 by referring to the statistical result of each measurement item. In this case, the database 2a may include an item for lot numbers of reagents used in the blood analyzer 1 in the test process, and the lot numbers of the used reagents may be displayed on the screens shown in FIGS. 28, 29.

On the screens shown in FIGS. 28, 29, a pull-down menu for displaying the statistical results for each device ID of the blood analyzer 1 may be disposed.

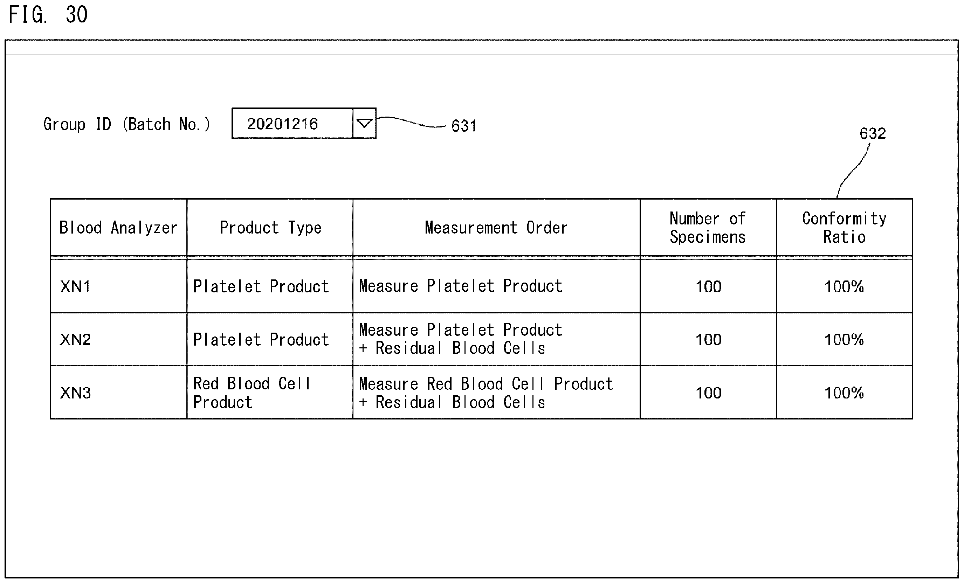

FIG. 30 schematically shows a screen displaying, for each device ID, quality control information based on the measurement results of blood products included in a group.

When the operator selects a group ID by operating a pull-down menu 631, quality control information of blood products corresponding to the group ID selected via the pull-down menu 631 is displayed for each device ID of the blood analyzer 1, in a list area 632. In the example shown in FIG. 30, items such as device ID, product type, measurement order, number of specimens, and conformity ratio are displayed in the list area 632.

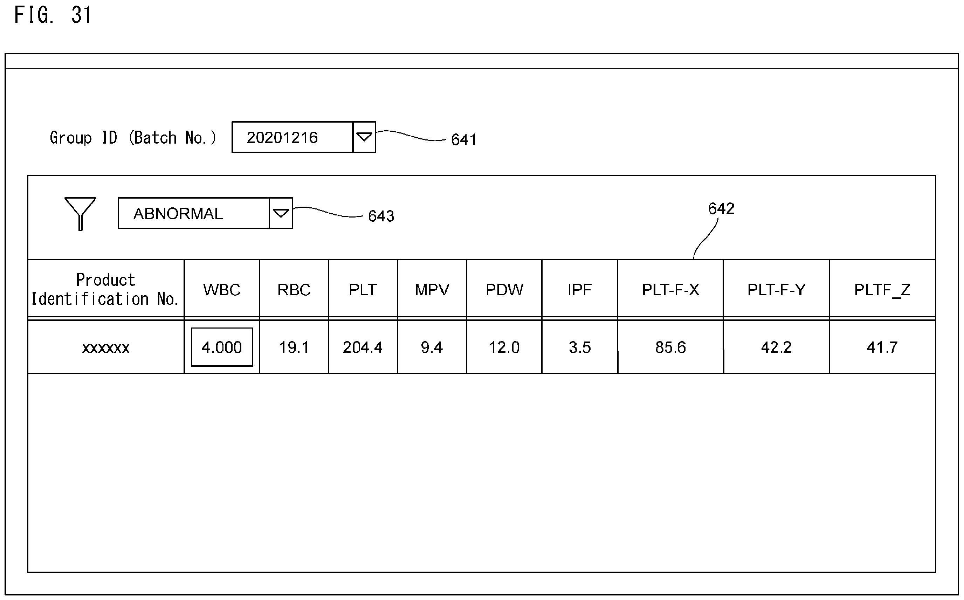

FIG. 31 schematically shows a screen displaying, according to a filter condition, quality control information based on the measurement results of blood products included in a group.

When the operator selects a group ID by operating a pull-down menu 641, the measurement results of blood products corresponding to the group ID selected via the pull-down menu 641 are displayed for each blood product in a list area 642. The operator can narrow down the blood products to be displayed in the list area 642 by operating a pull-down menu 643.

In the example shown in FIG. 31, an abnormal specimen (Abnormal) is selected in the pull-down menu 643, and therefore, only a blood product including an abnormal value in the measurement results is displayed in the list area 642. At this time, in order to make the abnormal value of the measurement item recognizable, the abnormal value is enclosed in a frame. Alternatively, the text color of the abnormal value may be changed to a text color (e.g., red) different from the normal text color.

### <Effects of management method, information processing apparatus, and information management system according to Embodiment 4>

The controller 201 of the information processing apparatus 2 further generates the quality control information for controlling the quality of the blood products as shown in FIGS. 28 to 31 on the basis of the measurement results and the other information on the blood products. According to this process, the quality of the blood products can be evaluated by effectively using the measurement results of the blood products and the other information on the blood products.

Generating quality control information includes: selecting at least one blood product from among a plurality of blood products registered in the database 2a on the basis of the other information on the blood products (step S41 in FIG. 27); and generating the quality control information on the basis of the measurement result of a specimen collected from the at least one blood product selected (step S42). According to this process, since the blood products to be evaluated are narrowed down, quality control is facilitated.

Generating quality control information includes: selecting a group consisting of a plurality of blood products (step S41 in FIG. 27); and generating the quality control information based on the measurement results of specimens collected from the respective blood products included in the selected group (step S42). According to this process, quality evaluation can be performed not for each blood product but for the selected group of blood products being a unit.

Selecting a group of blood products (step S41 in FIG. 27) includes selecting the group of blood products on the basis of a condition for specifying blood products produced in the same production cycle, through the screen shown in the upper part of FIG. 25. According to this process, a plurality of blood products produced in the same production cycle are selected to generate quality control information. Therefore, it is possible to: group, as one unit, a plurality of blood products for which the production process including, for example, the condition at which the blood products are produced (temperature, humidity, state of production facility, etc.) and the technique of the manufacturer involved in the production is assumed to be uniform; and evaluate the measurement results of the blood products for each unit. Therefore, in contrast to the case of evaluating the measurement results of individual blood products, quality control can be performed taking into account the production process.

Selecting a group of blood products (step S41 in FIG. 27) includes selecting the group of blood products on the basis of a condition for specifying blood products corresponding to specimens measured by the blood analyzer 1 in the same measurement cycle, via the screen shown in the lower part of FIG. 25. According to this process, a plurality of blood products measured in the same measurement cycle are selected to generate quality control information. Therefore, it is possible to: group, as one unit, a plurality of blood products for which it is assumed that the state of the blood analyzer 1 used for measurement of the blood products is uniform; and evaluate the measurement results of the blood products for each unit. Therefore, in contrast to the case of evaluating the measurement results of individual blood products, quality control can be performed taking into account the state of the blood analyzer 1.

As shown in FIGS. 28 to 30, the quality control information includes pieces of information (e.g., mean, standard deviation, coefficient of variation, minimum value, maximum value, conformity ratio, etc.) for evaluating the trends in the measurement results of the specimens collected from the group of blood products. According to this configuration, evaluation is performed not for each blood product but for the trends in the measurement results of the selected group of blood products, and therefore, quality control can be performed taking into account the trends of the blood product producing process and the state of the blood analyzer 1.

As shown in FIGS. 28, 29, the quality control information includes the statistical results (e.g., mean, standard deviation, coefficient of variation, etc.) of the measurement results of the specimens collected from a group of blood products. According to this configuration, by using the statistical results, the trends in the quality of the group of blood products can be quantitatively determined.

As shown in FIGS. 28, 29, the statistical results include statistical results (e.g., mean, standard deviation, coefficient of variation, etc.) corresponding to a plurality of measurement parameters (e.g., measurement items such as PLT and MPV) by the blood analyzer 1. According to this configuration, since the statistical results can be confirmed for each measurement parameter, trends in the quality of the blood product can be analyzed in detail.

The other information on the blood products includes information for specifying at least one of a blood product production cycle and a blood product measurement cycle. As the information for specifying the blood product production cycle and the blood product measurement cycle, "measurement date/time" by the blood analyzer 1 may be used, for example. In a factory where production and measurement of blood products are integrally performed, the blood product production cycle can be specified from the blood product measurement date/time. Therefore, for example, a plurality of blood products can be grouped as one unit, with each of time bands, such as morning, afternoon, and night, being one production cycle or one measurement cycle. For the same reason, information for specifying the blood product production cycle and the blood product measurement cycle may be the production date/time or the date/time when the measurement order was generated. According to the above configuration, the quality of the blood products grouped for each production cycle or measurement cycle can be controlled for each group unit.

The group unit effective for performing quality control may be defined according to not only the production cycle and the measurement cycle but also the blood collection facility, the manufacturer, the factory, the production device, and the like.

For example, in a case where the timing of arrival of blood at a factory varies depending on the distances from blood collection facilities to the factory and thereby the time band corresponding to the blood product production cycle varies depending on the timings, it is effective to perform grouping of blood products according to the blood collection facilities. For example, in a case where a manufacturer is changed for each production cycle in a factory, it is effective to perform grouping of blood products according to the manufacturers (manufacturer IDs). For example, in a case where pieces of information on blood products that are respectively produced in a plurality of factories at a plurality of separate locations are collectively managed, it is effective to perform grouping of blood products according to the factories (factory IDs). For example, in a factory where first to third centrifuges are used for producing blood products in the production cycle corresponding to the morning while fourth to sixth centrifuges are used in the production cycle corresponding to the afternoon, it is effective to perform grouping of blood products according to the identification information of the centrifuges being production devices. It is also effective to perform grouping of blood products according to not only the production devices in the factory but also devices installed in the blood collection facilities. For example, in a case where a plurality of blood collection machines for collecting blood from donors are installed in a blood collection facility and blood collection is performed by different blood collection machines according to work shifts in the blood collection facility, it is effective to perform grouping of blood products according to identification information of the blood collection machines.

The other information on the blood product includes information on the time when production of the blood product or measurement of the specimen collected from the blood product was performed. The time when production of the blood product was performed is the reception date/time, the production date/time, or the like, for example. The time when measurement of the specimen collected from the blood product was performed is the measurement date/time, for example. According to this configuration, blood products can be grouped based on time, and therefore can be sorted for each timing or time band when blood of donors arrives at the factory.

Selecting at least one blood product includes selecting blood products produced in a certain time band in one day, through the screen shown in FIG. 25. For example, in a case where quality control is performed on a daily basis, shipping of blood products corresponding to the first arrival of blood bags in the morning is not performed until the night when the test for the day is completed, which impedes quick shipping. However, according to the above process, in contrast to the daily-basis quality control, quality control can be quickly performed after production of blood products and the blood products can be quickly shipped.

The controller 201 of the information processing apparatus 2 receives setting of a condition for selecting at least one blood product, through the screen shown in FIG. 25. According to this process, the blood product can be selected based on the condition according to the operation in the factory.

The condition for selecting at least one blood product is a condition for forming a batch consisting of one or a plurality of blood products. A batch means a unit of blood products that are produced together in the same production cycle and are measured in the same measurement cycle. According to this configuration, even blood products whose raw materials are not homogeneous can be subjected to batch management according to the factory.

The controller 201 of the information processing apparatus 2 receives designation of any batch among a plurality of batches formed according to the condition for selecting at least one blood product, through the pull-down menus regarding group IDs shown in FIGS. 28 to 31 (step S41 in FIG. 27), and displays quality control information corresponding to the designated batch as shown in the list area and the graph area on the screens shown in FIGS. 28 to 31 (step S43). According to this process, the operator can easily confirm information of a specific batch.

As shown in the list area 632 in FIG. 30, the quality control information includes information on the conformity ratio of the blood products in the batch. Since the conformity ratio indicating the ratio of blood products conforming to a quality standard to blood products included in one batch, is displayed, it is possible to judge what percentage of blood products in the batch has abnormal measurement results. Therefore, the conformity ratio is a useful index for judging whether or not a problem has occurred in the blood product producing process or the state of the blood analyzer 1.

The controller 201 of the information processing apparatus 2 receives approval for blood products in batch units via the validation button 624 shown in FIGS. 28, 29. According to this process, judgement for shipping in batch units can be easily performed.

### <Embodiment 5>

In order to assure test accuracy for blood products, a quality control sample (hereinafter, referred to as "QC sample") is measured at a predetermined timing (e.g., morning, noon, or evening) by the blood analyzer 1, thereby generating a measurement result of the QC sample. In Embodiment 5, information related to the measurement result based on a single measurement of the QC sample (hereinafter, referred to as "quality control information") is associated with product identification information or a group ID. The operator can confirm the state of the blood analyzer 1 at the time of measurement with reference to the quality control information corresponding to one blood product or one group of blood products.

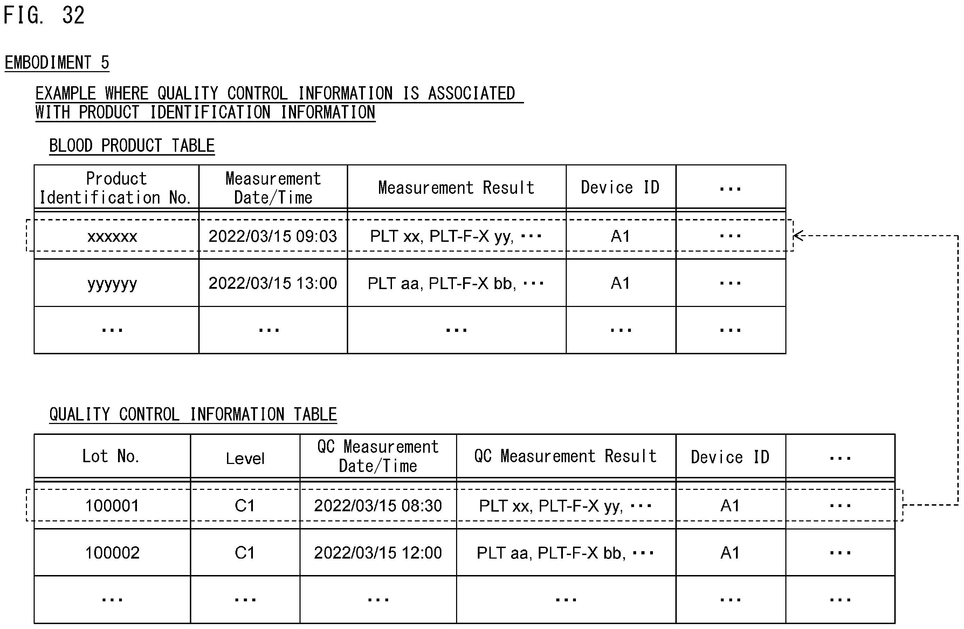

FIG. 32 shows an example in which quality control information is associated with product identification information, according to Embodiment 5.

As shown in the upper part of FIG. 32, in this case, as in the above embodiments, the database 2a includes a blood product table for storing information on blood products including measurement results, in association with product identification information. In addition, as shown in the lower part of FIG. 32, the database 2a further includes a quality control information table for storing, as quality control information, lot No., level, QC measurement date/time, device ID of a blood analyzer 1 performing QC measurement, and the like for each measurement of the QC sample. The lot number is an ID assigned for each QC sample, and the level is the concentration of a component contained in the QC sample.

For example, when the operator inputs an instruction for displaying quality control information corresponding to a target blood product, the controller 201 extracts, from the quality control information table, quality control information which includes the same device ID as that of the target blood product and whose QC measurement date/time is closest to the measurement date/time of the target blood product. In the example of FIG. 32, a blood product in the first line of the blood product table is a target to be displayed, and the first line of the quality control information table is extracted as information on quality control performed immediately before the measurement date/time of this blood product. The controller 201 displays information on the blood product, with the extracted quality control information being associated with the product identification information of the target blood product.

Association between the product identification information and the quality control information may not necessarily be performed at the time of displaying the information on the blood product, and may be performed in advance by the controller 201 at a predetermined timing (e.g., morning, noon, or evening). In this case, the database 2a is provided with another table for associating the product identification information with the quality control information, and the controller 201 stores, in the other table, association between the product identification information identifying the blood product and the information (e.g., lot No. and QC measurement date/time) specifying the quality control information, at the predetermined timing.

Meanwhile, product identification information of a group of blood products having the same group ID may be associated with quality control information. In this case, for example, a common group ID is assigned to product identification information of a group of blood products subjected to measurement in one measurement cycle. Measurement of the QC sample is not performed during the period of the measurement cycle, and is performed before and after the measurement cycle. The results of QC sample measurements performed immediately before and after the measurement cycle are associated, as the quality control information, with the product identification information of the blood products measured in the measurement cycle, i.e., with the product identification information of the group of blood products to which the common group ID is assigned.

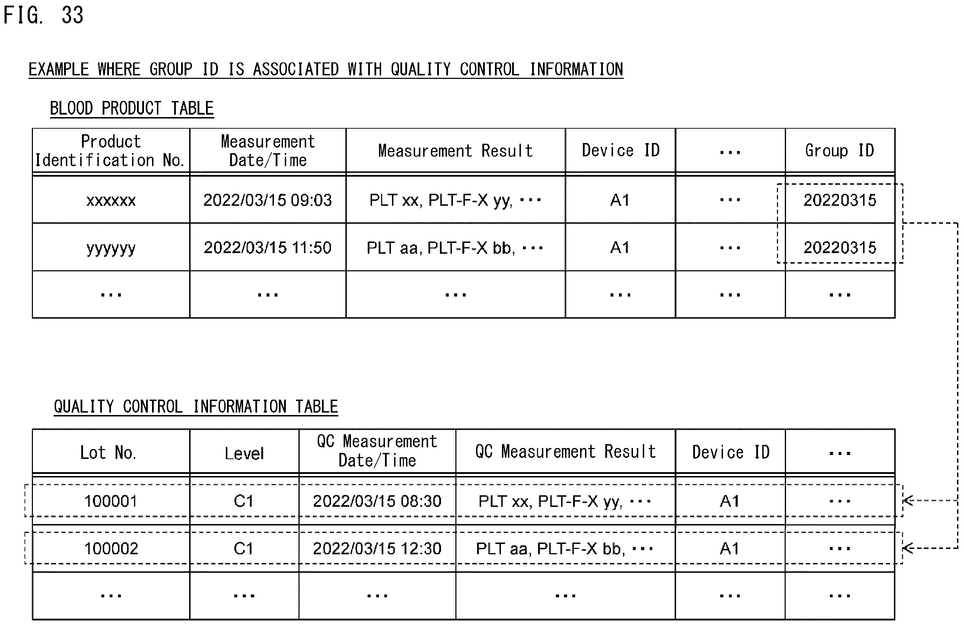

FIG. 33 shows an example in which a group ID is associated with quality control information.

In this case, in contrast to FIG. 32, the blood product table in the database 2a further includes an item of group ID. Further, in the example shown in FIG. 33, the same group ID, "20220315", is assigned to blood products measured by the blood analyzer 1 whose device ID is "A1", i.e., the blood analyzer 1 to which the same measurement cycle is applied.

For example, when the operator inputs an instruction for displaying quality control information corresponding to a target group of blood products, the controller 201 extracts, from the quality control information table, pieces of quality control information which include the same device ID as that of the target group of blood products and whose QC measurement dates/times are immediately before and after the time band of measurement for the target group. In the example shown in FIG. 33, a group ID of "20220315" is a display target, and the first and second lines of the quality control information table are extracted as pieces of information on quality controls performed immediately before and after the time band of measurement for the target group. The controller 201 associates the extracted quality control information with the group ID of the target blood products, and displays information on the group of blood products.

Association between the group ID and the quality control information may not necessarily be performed at the time of displaying the information on the blood products, and may be performed in advance by the controller 201 at a predetermined timing (e.g., morning, noon, or evening). In this case, the database 2a is provided with another table for associating the group ID with the quality control information, and the controller 201 stores, in the other table, association between the group ID specifying the group and the information (e.g., lot No. and QC measurement date/time) specifying the quality control information, at the predetermined timing.

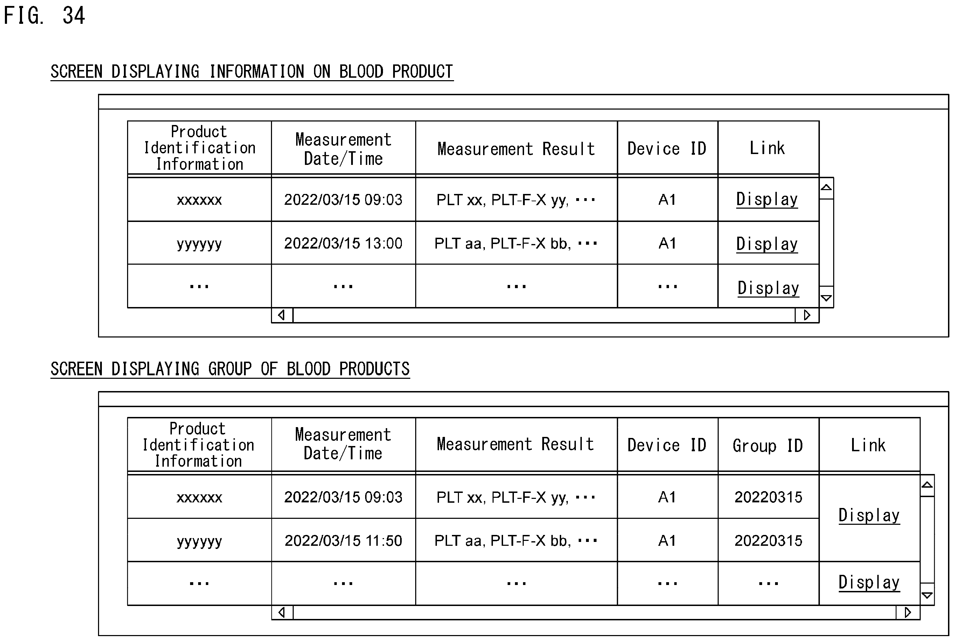

The upper and lower parts of FIG. 34 schematically show a screen displaying information on blood products and a screen displaying groups of blood products, respectively. The two screens shown in FIG. 34 are displayed on the display 203 by the controller 201 in response to a display instruction that is inputted by the operator operating the input unit 204.

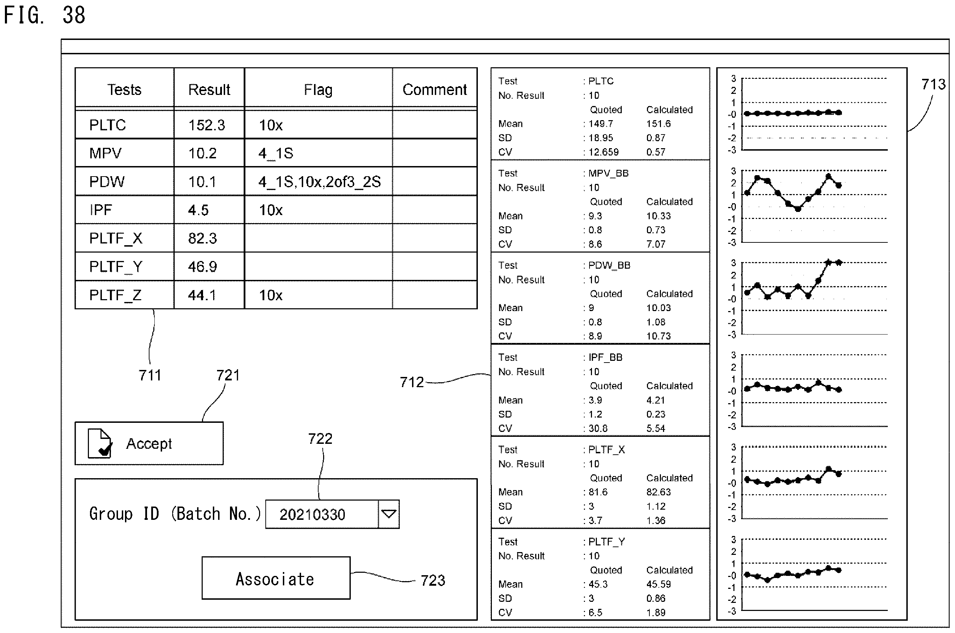

As shown in the upper part of FIG. 34, on the screen that displays the information on blood products, a link item is assigned for each piece of product identification information. As shown in the lower part of FIG. 34, on the screen that displays the groups of blood products, a link item is assigned for each group ID. When the operator clicks "display" under the link item, a screen (e.g., the same screen as shown in FIG. 38 described later) that displays details of quality control information corresponding to the blood product or the group is displayed on the display 203. When there are a plurality of pieces of quality control information, a screen for selecting display of these pieces of quality control information is displayed. Thus, the link for displaying the quality control information for each blood product or each group enables smooth confirmation of the quality control information.

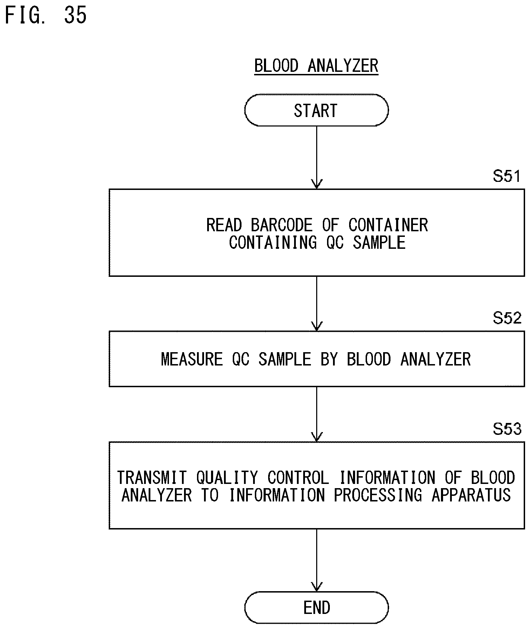

FIG. 35 is a flowchart showing a process performed by the blood analyzer 1.

In step S51, the controller 121 of the blood analyzer 1 instructs the measurement controller 111 to read identification information from a barcode on a container containing a QC sample, and the measurement controller 111 drives the reader 113. The read identification information includes the lot No. and the level of the QC sample contained in the container. In step S52, the controller 121 of the blood analyzer 1 measures the QC sample with the blood analyzer 1 to obtain a measurement result, as in the case of the blood product. In step S53, the controller 121 transmits, to the information processing apparatus 2, quality control information including lot No., level, QC measurement date/time, QC measurement result, and device ID.

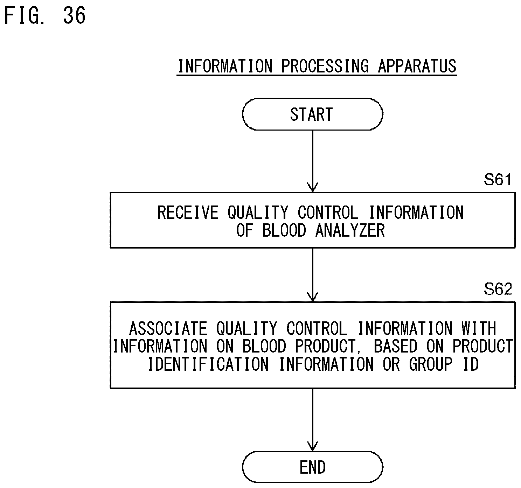

FIG. 36 is a flowchart showing a process performed by the information processing apparatus 2.

In step S61, the controller 201 of the information processing apparatus 2 receives the quality control information transmitted in step S53 in FIG. 35, and stores the received quality control information into the quality control information table of the database 2a. In step S62, the controller 201 associates the quality control information with information on a blood product (the measurement result and the other information on the blood product) on the basis of the product identification information or the group ID. As described above, association of the quality control information may be performed when displaying the information on the blood product, or may be performed at a predetermined timing.

Association of the quality control information may not necessarily be automatically performed by the controller 201 as described above, and may be manually performed by the operator.

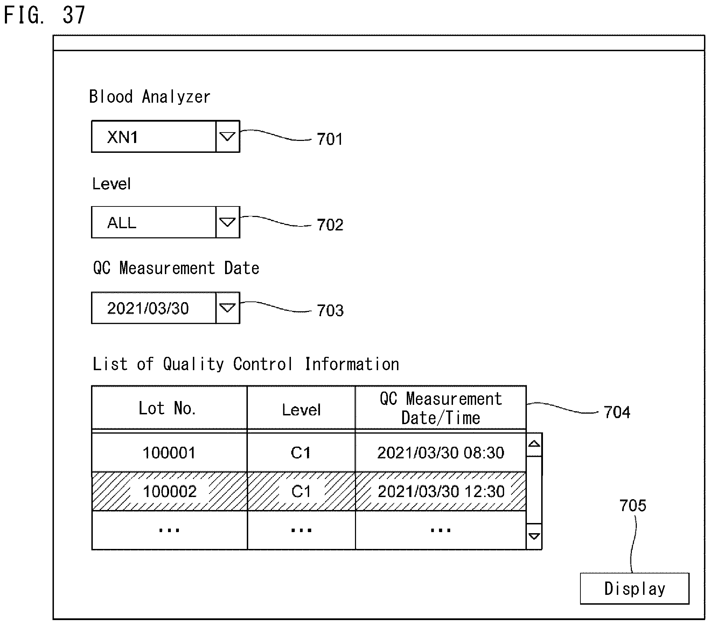

With reference to FIGS. 37 to 39, how the operator manually performs association of quality control information will be described. Each of screens shown in FIGS. 37 to 39 is displayed on the display 203 by the controller 201 in response to a display instruction that is inputted by the operator operating the input unit 204.

In the following example, a group ID of "20210330" is set in advance on blood products which were measured in the afternoon of March 30, 2021 and for which a blood analyzer 1 having a device ID of "XN1" was used. The operator associates a group of blood products that were produced in the morning of March 30, 2021 and measured in the afternoon of that day, with quality control information. In addition, the operator associates the group of blood products with only quality control information having appropriate measurement results, among measurement results of the QC sample measured immediately before and after the measurement cycle for the group of blood products.

FIG. 37 schematically shows a screen for displaying a list of quality control information.

The operator selects a device ID of a blood analyzer 1 by operating a pull-down menu 701, selects a level of QC sample by operating a pull-down menu 702, and selects a QC measurement date by operating a pull-down menu 703. When the pull-down menus 701 to 703 are operated, corresponding quality control information is extracted from the quality control information table on the basis of the selected device ID, level, and QC measurement date, and the extracted quality control information is displayed in a list area 704. In this example, the operator selects the second line in the list area 704, as quality control information corresponding to the group of blood products measured in the afternoon of March 30, 2021. When the operator operates a display button 705, details of the quality control information selected in the list area 704 are displayed on the display 203 of the information processing apparatus 2 as shown in FIG. 38.

FIG. 38 schematically shows a screen showing the details of the quality control information.

In a list area 711, a detail display area 712, and a graph area 713, the details of the quality control information selected in FIG. 37 are displayed. Specifically, in the list area 711, a measurement result, a flag, and a comment are displayed for each measurement item. In the detail display area 712, statistical information obtained by aggregating the quality control information and previous quality control information is displayed for each measurement item. In the graph area 713, change over time in the measurement results is represented by graphs.

When the operator determines that the quality control information is appropriate with reference to the list area 711, the detail display area 712, and the graph area 713, the operator operates an acceptance button 721. Furthermore, the operator selects a group ID by operating a pull-down menu 722. In this example, the operator selects "20210330" as a group ID in order to associate the group of blood products measured in the afternoon of March 30, 2021 with the quality control information shown in FIG. 38. When the operator operates an association button 723, the group ID selected via the pull-down menu 722 is associated with the quality control information shown in FIG. 38.

Thus, the operator approves the quality control information, and associates the target group ID with the approved quality control information. The controller 201 of the information processing apparatus 2 stores this association in the table in the database 2a that manages this association.

Usually, in order to assure reliability of measurement results of a group of blood products measured in a measurement cycle, it is preferable that pieces of quality control information immediately before and after the measurement cycle are both appropriate. Therefore, one group of blood products is preferably associated with both pieces of quality control information that are immediately before and after the measurement cycle, through the process shown in FIG. 38. This enables confirmation that measurement for the target group (batch) has been performed with high reliability.

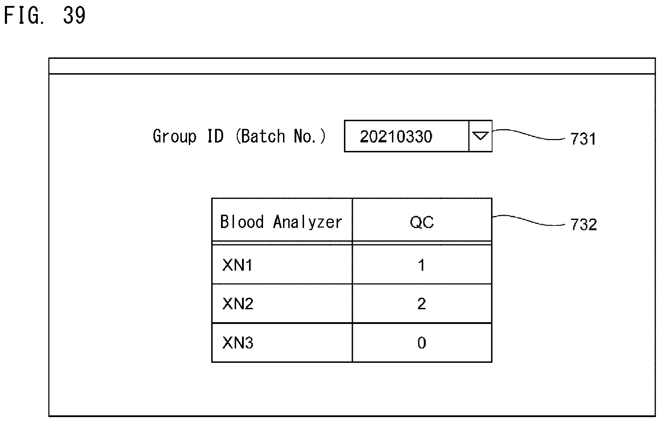

FIG. 39 schematically shows a screen for confirming the number of pieces of approved quality control information associated with a group ID.

When the operator selects a group ID by operating a pull-down menu 731, the number of pieces of approved quality control information is displayed for each device ID of a blood analyzer 1 in a list area 732.

In this example, the blood products in the target group (batch) are separately measured by three blood analyzers 1 whose device IDs are XN1, XN2, XN3, in one measurement cycle. Association of the target group (batch) with quality control information is performed for each blood analyzer 1 through the screens shown in FIGS. 37, 38. Therefore, FIG. 39 shows the numbers (1, 2, 0) of pieces of approved quality control information corresponding to the blood analyzers 1 whose device IDs are XN1, XN2, XN3, respectively.

As described above, usually, it is preferable that one measurement cycle is associated with two pieces of approved quality control information. Therefore, it can be said that reliability of measurement performed by the blood analyzer 1 (device ID: XN2) having two pieces of approved quality control information is high.

### <Effects of management method, information processing apparatus, and information management system according to Embodiment 5>

The controller 201 of the information processing apparatus 2 obtains information on quality control for the blood analyzer 1 (step S61 in FIG. 36), and associates the information on quality control with the measurement result and the other information on the blood product on the basis of the product identification information used as specimen identification information (step S62). According to this process, when evaluating the quality or the like of the blood product, information on quality control is also effectively used, whereby it is possible to take into account whether or not the state of the blood analyzer 1 at the time of measuring the blood product was normal.

### <Embodiment 6>

In the above embodiments, information on blood products is stored only in the database 2a of the information processing apparatus 2. In Embodiment 6, part of the information on blood products is also stored in a database 6a of an intermediate server 6.

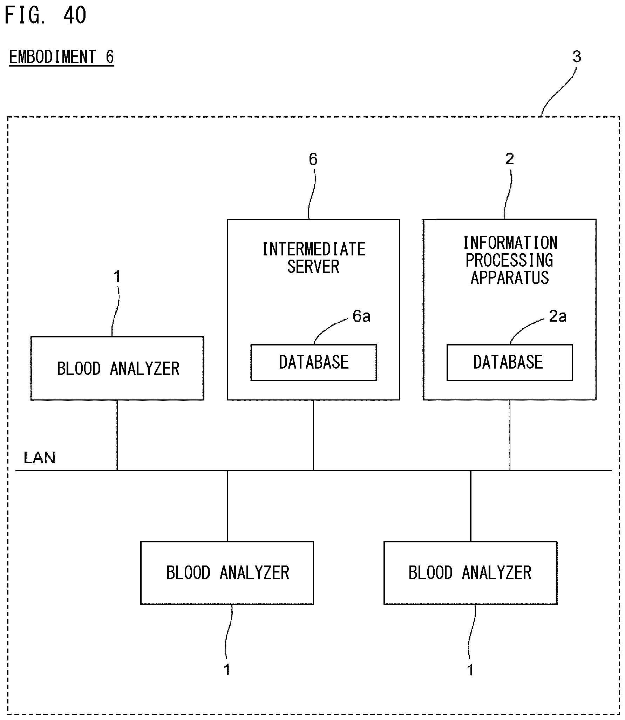

FIG. 40 schematically shows connection of devices according to Embodiment 6.

In FIG. 40, in contrast to FIG. 19, one intermediate server 6 is added in the factory. Three blood analyzers 1, one information processing apparatus 2, and one intermediate server 6 constitute an information management system 3. The three blood analyzers 1, the one information processing apparatus 2, and the one intermediate server 6 are connected to a LAN in the factory. This connection allows the three blood analyzers 1, the information processing apparatus 2, and the intermediate server 6 to communicate with each other.

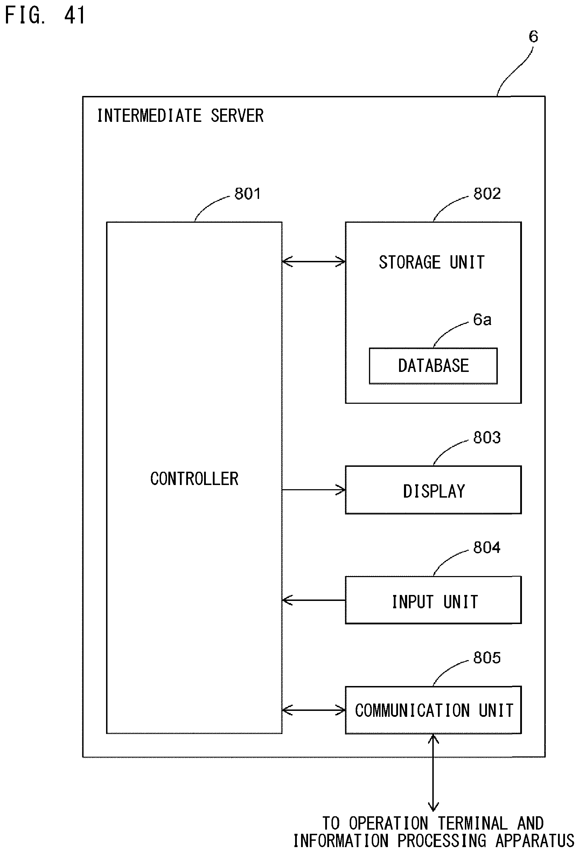

FIG. 41 is a block diagram showing the configuration of the intermediate server 6.

The intermediate server 6 includes a controller 801, a storage unit 802, a display 803, an input unit 804, and a communication unit 805. The storage unit 802 has the database 6a stored therein. The controller 801, the storage unit 802, the display 803, the input unit 804, and the communication unit 805 of the intermediate server 6 are identical in configuration to the controller 201, the storage unit 202, the display 203, the input unit 204, and the communication unit 205 of the information processing apparatus 2, respectively.

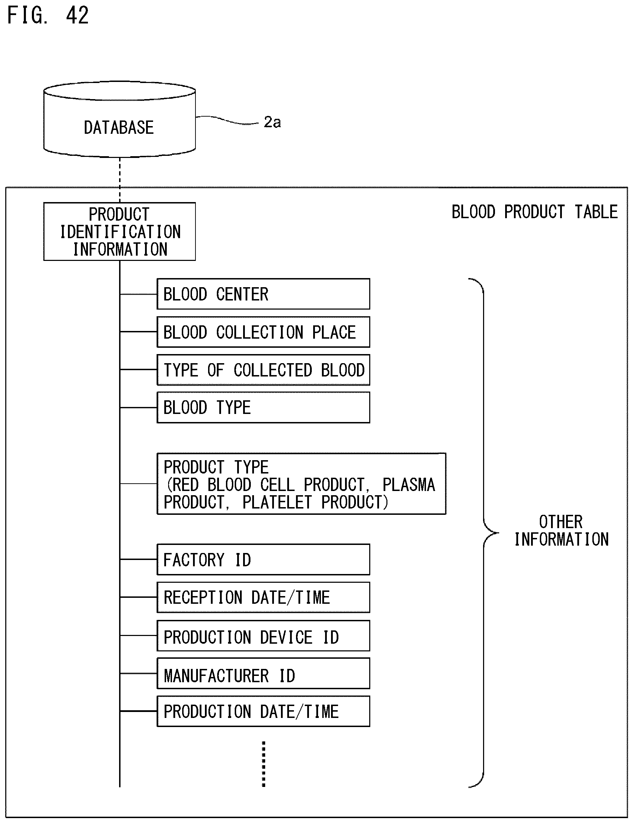

FIG. 42 schematically shows items stored in the database 6a.

The database 6a stores therein information on a blood product other than information regarding measurement. Specifically, the database 6a includes items such as product identification information, blood center, blood collection facility, type of collected blood, blood type, product type, factory ID, reception date/time, production device ID, manufacturer ID, and production date/time, and does not include items such as measurement order, measurement result, measurement date/time, and device ID.

When the operator inputs product identification information and other information on the blood product by using the operation terminal in the production process, these pieces of information are transmitted to the intermediate server 6 and stored in the database 6a. In the test process, the information stored in the database 6a of the intermediate server 6 is transmitted to the information processing apparatus 2 and stored in the database 2a. Thereafter, the measurement result or the like obtained through measurement by the blood analyzer 1 are transmitted to the information processing apparatus 2 and stored in the database 2a. Thus, the information finally stored in the database 2a is identical to that of the above embodiments.

### <Effects of management method, information processing apparatus, and information management system according to Embodiment 6>

As shown in FIG. 40, the database in which the information on the blood product is stored in association with the product identification information includes: the database 2a (first database) that stores therein the measurement result in association with the product identification information; and the database 6a (second database) that stores therein the other information in association with the product identification information. This configuration allows distribution of the load to the databases.

### <Modification of Embodiment 6>

In Embodiment 6, all the information stored in the database 6a of the intermediate server 6 is finally stored in the database 2a of the information processing apparatus 2. However, not all the information in the database 6a needs to be transmitted to the information processing apparatus 2. For example, all the information in the database 6a may not be transmitted to the information processing apparatus 2.

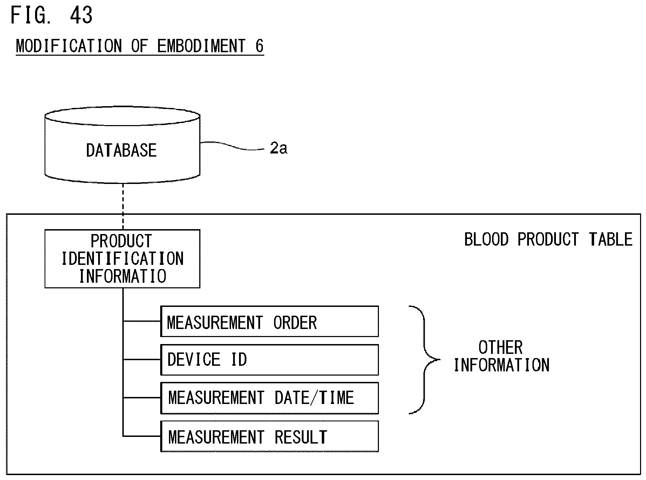

FIG. 43 schematically shows items stored in the database 2a, according to a modification of Embodiment 6. In this modification, in contrast to Embodiment 6, the database 2a includes only the items regarding measurement, such as measurement order, measurement result, measurement date/time, and device ID. The configuration of the database 6a is identical to that shown in FIG. 42. In this modification, the information stored in the database 6a is not transmitted to the information processing apparatus 2.

In this case, when the operator inputs an instruction for causing the display 203 to display information on a blood product, by operating the information processing apparatus 2, the controller 201 of the information processing apparatus 2 transmits a transmission request to the intermediate server 6 in order to obtain information on the blood product other than the items shown in FIG. 43. In response to the transmission request, the controller 801 of the intermediate server 6 obtains necessary information from the database 6a, and transmits the obtained information to the information processing apparatus 2. On the basis of the information stored in the database 2a and the information received from the intermediate server 6, the controller 201 of the information processing apparatus 2 displays the information on the blood product on the display 203, as in the above embodiments.

### <Embodiment 7>

In the above embodiments, the information processing apparatus 2 receives the specimen measurement result from the blood analyzer 1. Meanwhile, in Embodiment 7, a blood analyzer 7 has the function of the information processing apparatus 2.

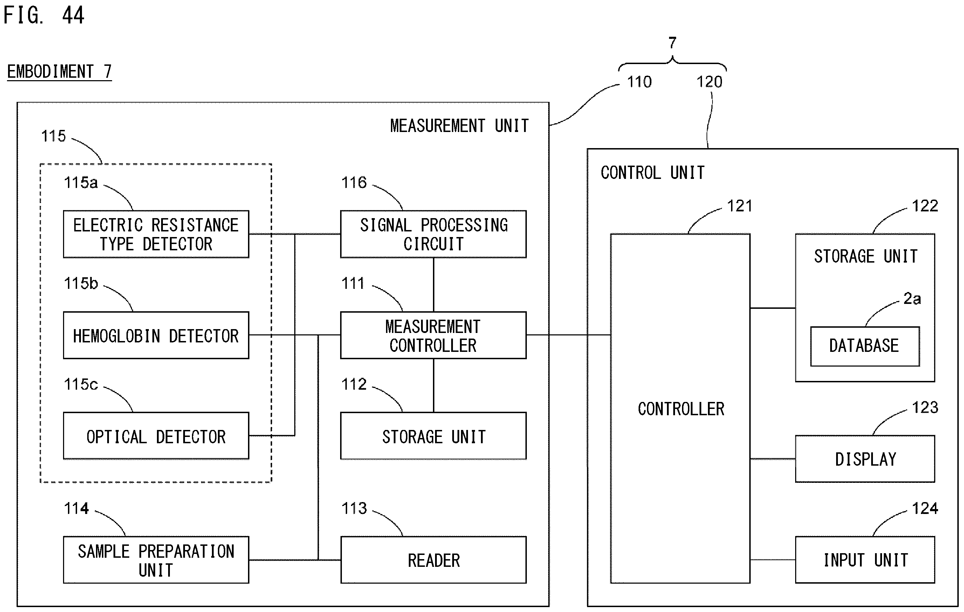

FIG. 44 is a block diagram showing the configuration of the blood analyzer 7 according to Embodiment 7.

In FIG, 44, in contrast to FIG. 5, the storage unit 122 has the database 2a stored therein. The communication unit 125 is removed.

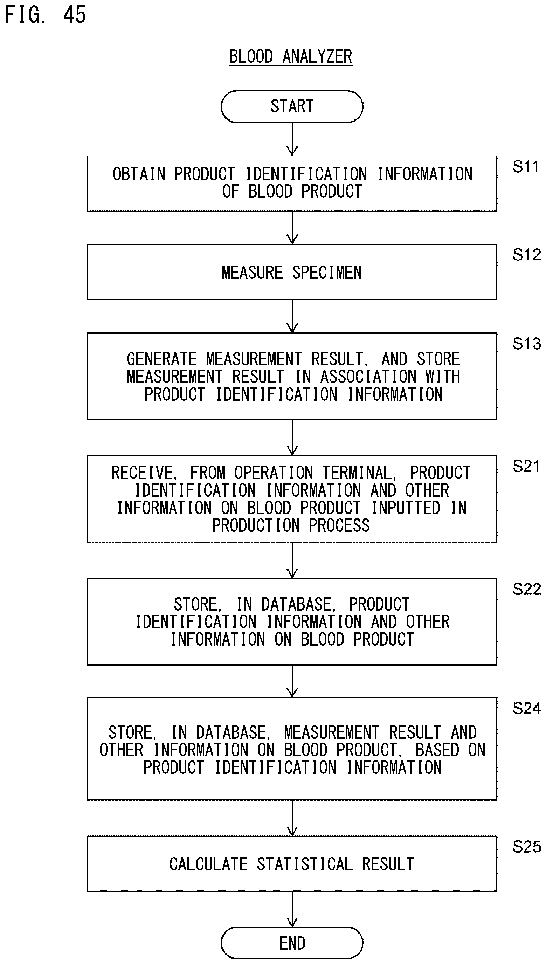

FIG. 45 is a flowchart showing a process performed by the blood analyzer 7 according to Embodiment 7. In the storage unit 122, a program for executing the processes in steps S21, S22, S24, and S25 shown in FIG. 8 is stored in addition to a program for executing the processes in steps S11 to S13 shown in FIG. 7. When the controller 121 executes the programs stored in the storage unit 122, the respective processes shown in FIG. 45 are executed. In FIG. 45, in contrast to FIGS. 7, 8, the controller 121 executes all the processes. In addition, steps S14 and S23 regarding transmission/reception of the specimen identification information and the measurement result are deleted.

### <Effects of management method and blood analyzer according to Embodiment 7>

The blood analyzer 7 has the function of the information processing apparatus 2. Therefore, a computer other than the blood analyzer 7 and a communication infrastructure for communicably connecting the blood analyzer 7 and a computer can be dispensed with, thereby simplifying the hardware configuration.

Various modifications can be made as appropriate to the embodiments of the present invention, without departing from the scope of the technological idea defined by the claims.

### [Remarks]

The present disclosure includes the following items 1-29.

Item 1: A method for managing information on a blood product with a computer, comprising:
obtaining a measurement result of a specimen, collected from the blood product, which is measured by a blood analyzer with product identification information of the blood product being used as specimen identification information of the specimen, and the specimen identification information; and
in a database in which information on the blood product is stored in association with the product identification information, associating the measurement result with other information on the blood product other than the measurement result, on the basis of the product identification information used as the specimen identification information.

Item 2: The management method of item 1, wherein
the product identification information is identification information assigned to a blood bag that contains blood collected from a donor.

Item 3: The management method of item 1, wherein
the product identification information includes a number obtained by encoding at least a part of the other information on the blood product.

Item 4: The management method of item 1, wherein
the other information on the blood product includes information regarding at least one of property of the blood product, a production process for the blood product, and a measurement process for the blood product.

Item 5: The management method of item 4, wherein
the blood analyzer includes a plurality of blood analyzers, and
the information regarding the measurement process for the blood product includes information for specifying a blood analyzer that has measured the specimen.

Item 6: The management method of item 1, further comprising:
selecting at least one blood product from among a plurality of blood products registered in the database on the basis of the other information on the blood product; and
generating information by aggregating the measurement results of specimens collected from the at least one blood product selected.

Item 7: The management method of item 1, further comprising
generating quality control information for managing quality of the blood product on the basis of the measurement result and the other information on the blood product.

Item 8: The management method of item 7, wherein
the generating of the quality control information includes:
selecting at least one blood product from among a plurality of blood products registered in the database on the basis of the other information on the blood product; and
generating the quality control information on the basis of the measurement result of a specimen collected from the at least one blood product selected.

Item 9: The management method of item 8, wherein
the generating of the quality control information includes:
selecting a group consisting of a plurality of blood products; and
generating the quality control information based on the measurement results of specimens collected from the respective blood products included in the selected group of blood products.

Item 10: The management method of item 9, wherein
the selecting of the group of blood products includes selecting the group of blood products on the basis of a condition for specifying blood products produced in the same production cycle.

Item 11: The management method of item 9, wherein
the selecting of the group of blood products includes selecting the group of blood products on the basis of a condition for specifying blood products corresponding to specimens measured by the blood analyzer in the same measurement cycle.

Item 12: The management method of item 9, wherein
the quality control information includes information for evaluating trends in the measurement results of the specimens collected from the group of blood products.

Item 13: The management method of item 9, wherein
the quality control information includes a statistical result of the measurement results of the specimens collected from the group of blood products.

Item 14: The management method of item 13, wherein
the statistical result includes statistical results respectively corresponding to a plurality of measurement parameters of measurement by the blood analyzer.

Item 15: The management method of item 8, wherein
the other information on the blood product includes information for specifying at least one of a blood product production cycle and a blood product measurement cycle.

Item 16: The management method of item 15, wherein
the other information on the blood product includes information regarding a time when production of the blood product or measurement of a specimen collected from the blood product was performed.

Item 17: The management method of item 16, wherein
the selecting of the at least one blood product includes selecting blood products produced in a time band corresponding to a part of one day.

Item 18: The management method of item 8, further comprising
receiving setting of a condition for selecting the at least one blood product.

Item 19: The management method of item 18, wherein
the condition is a condition for forming a batch consisting of one or a plurality of blood products.

Item 20: The management method of item 19, further comprising:
receiving designation of any batch among a plurality of batches formed according to the condition; and
displaying the quality control information corresponding to the designated batch.

Item 21: The management method of item 20, wherein
the quality control information includes information regarding a conformity ratio of the blood products in the batch.

Item 22: The management method of item 20, further comprising
receiving an approval for the blood products in each batch.

Item 23: The management method of item 1, further comprising:
obtaining information regarding quality control of the blood analyzer; and
associating the information regarding the quality control with the measurement result and the other information on the blood product, on the basis of the product identification information used as the specimen identification information.

Item 24: The management method of item 1, wherein
the database includes:
a first database that stores therein the measurement result in association with the product identification information; and
a second database that stores therein the other information in association with the product identification information.

Item 25: The management method of item 1, wherein
the computer is communicably connected to the blood analyzer, and
the obtaining of the measurement result of the specimen and the specimen identification information includes obtaining the measurement result of the specimen and the specimen identification information from the blood analyzer.

Item 26: An information processing apparatus comprising:
a storage unit; and
a controller programmed to store information on a blood product into the storage unit, wherein
the controller is programmed to
   obtain, from a blood analyzer, a measurement result of a specimen, collected from the blood product, which is measured with product identification information of the blood product being used as specimen identification information of the specimen, and the specimen identification information, and
   in a database, in the storage unit, in which information on the blood product is stored in association with the product identification information, associate the measurement result with other information on the blood product other than the measurement result, on the basis of the product identification information used as the specimen identification information.

Item 27: An information management system comprising:
a blood analyzer capable of measuring a specimen collected from a blood product, with product identification information of the blood product being used as specimen identification information of the specimen; and
an information processing apparatus communicably connected to the blood analyzer, wherein
the information processing apparatus includes
   a storage unit, and
   a controller programmed to store information on the blood product into the storage unit, and
the controller is programmed to
   obtain, from the blood analyzer, a measurement result of the specimen and the specimen identification information, and
   in a database, in the storage unit, in which information on the blood product is stored in association with the product identification information, associate the measurement result with other information on the blood product other than the measurement result, on the basis of the product identification information used as the specimen identification information.

Item 28: An information management system comprising:
a blood analyzer configured to measure a specimen collected from a blood product; and
an information processing apparatus communicably connected to the blood analyzer, wherein
the blood analyzer obtains product identification information of the blood product, measures the specimen, and automatically transmits a measurement result of the specimen and the product identification information of the blood product to the information processing apparatus,
the information processing apparatus includes
   a storage unit, and
   a controller programmed to store information on the blood product into the storage unit, and
the controller is programmed to
   receive, from the blood analyzer, the measurement result of the specimen and the product identification information of the blood product, and
   in a database, in the storage unit, in which the information on the blood product is stored in association with the product identification information, automatically associate the received measurement result with other information on the blood product other than the measurement result, on the basis of the received product identification information.

Item 29: A blood analyzer comprising:
a detector configured to detect a predetermined component from a specimen collected from a blood product;
a reader configured to read product identification information of the blood product as specimen identification information of the specimen; and
a storage unit,
the blood analyzer being configured to
generate a measurement result of the specimen on the basis of a detection result of the detector, and
in a database, in the storage unit, in which information on the blood product is stored in association with the product identification information, associate the measurement result with other information on the blood product other than the measurement result, on the basis of the product identification information used as the specimen identification information.

## Claims

1. A method for managing information on a blood product with a computer, comprising:
obtaining a measurement result of a specimen, collected from the blood product, which is measured by a blood analyzer with product identification information of the blood product being used as specimen identification information of the specimen, and the specimen identification information; and
in a database in which information on the blood product is stored in association with the product identification information, associating the measurement result with other information on the blood product other than the measurement result, on the basis of the product identification information used as the specimen identification information.

2. The management method of claim 1, wherein
the product identification information is identification information assigned to a blood bag that contains blood collected from a donor.

3. The management method of claim 1, wherein
the product identification information includes a number obtained by encoding at least a part of the other information on the blood product.

4. The management method of claim 1, wherein
the other information on the blood product includes information regarding at least one of property of the blood product, a production process for the blood product, and a measurement process for the blood product.

5. The management method of claim 4, wherein
the blood analyzer includes a plurality of blood analyzers, and
the information regarding the measurement process for the blood product includes information for specifying a blood analyzer that has measured the specimen.

6. The management method of claim 1, further comprising:
selecting at least one blood product from among a plurality of blood products registered in the database on the basis of the other information on the blood product; and
generating information by aggregating the measurement results of specimens collected from the at least one blood product selected.

7. The management method of claim 1, further comprising
generating quality control information for managing quality of the blood product on the basis of the measurement result and the other information on the blood product.

8. The management method of claim 7, wherein
the generating of the quality control information includes:
selecting at least one blood product from among a plurality of blood products registered in the database on the basis of the other information on the blood product; and
generating the quality control information on the basis of the measurement result of a specimen collected from the at least one blood product selected.

9. The management method of claim 8, wherein
the generating of the quality control information includes:
selecting a group consisting of a plurality of blood products; and
generating the quality control information based on the measurement results of specimens collected from the respective blood products included in the selected group of blood products.

10. The management method of claim 9, wherein
the selecting of the group of blood products includes selecting the group of blood products on the basis of a condition for specifying blood products produced in the same production cycle.

11. The management method of claim 9, wherein
the selecting of the group of blood products includes selecting the group of blood products on the basis of a condition for specifying blood products corresponding to specimens measured by the blood analyzer in the same measurement cycle.

12. The management method of claim 9, wherein
the quality control information includes information for evaluating trends in the measurement results of the specimens collected from the group of blood products.

13. The management method of claim 9, wherein
the quality control information includes a statistical result of the measurement results of the specimens collected from the group of blood products.

14. The management method of claim 13, wherein
the statistical result includes statistical results respectively corresponding to a plurality of measurement parameters of measurement by the blood analyzer.

15. The management method of claim 8, wherein
the other information on the blood product includes information for specifying at least one of a blood product production cycle and a blood product measurement cycle.

16. The management method of claim 15, wherein
the other information on the blood product includes information regarding a time when production of the blood product or measurement of a specimen collected from the blood product was performed.

17. The management method of claim 16, wherein
the selecting of the at least one blood product includes selecting blood products produced in a time band corresponding to a part of one day.

18. The management method of claim 8, further comprising
receiving setting of a condition for selecting the at least one blood product.

19. The management method of claim 18, wherein
the condition is a condition for forming a batch consisting of one or a plurality of blood products.

20. The management method of claim 19, further comprising:
receiving designation of any batch among a plurality of batches formed according to the condition; and
displaying the quality control information corresponding to the designated batch.

21. The management method of claim 20, wherein
the quality control information includes information regarding a conformity ratio of the blood products in the batch.

22. The management method of claim 20, further comprising
receiving an approval for the blood products in each batch.

23. The management method of claim 1, further comprising:
obtaining information regarding quality control of the blood analyzer; and
associating the information regarding the quality control with the measurement result and the other information on the blood product, on the basis of the product identification information used as the specimen identification information.

24. The management method of claim 1, wherein
the database includes:
a first database that stores therein the measurement result in association with the product identification information; and
a second database that stores therein the other information in association with the product identification information.

25. The management method of claim 1, wherein
the computer is communicably connected to the blood analyzer, and
the obtaining of the measurement result of the specimen and the specimen identification information includes obtaining the measurement result of the specimen and the specimen identification information from the blood analyzer.

26. An information processing apparatus comprising:
a storage unit; and
a controller programmed to store information on a blood product into the storage unit, wherein
the controller is programmed to
obtain, from a blood analyzer, a measurement result of a specimen, collected from the blood product, which is measured with product identification information of the blood product being used as specimen identification information of the specimen, and the specimen identification information, and
in a database, in the storage unit, in which information on the blood product is stored in association with the product identification information, associate the measurement result with other information on the blood product other than the measurement result, on the basis of the product identification information used as the specimen identification information.
